(19) Europäisches Patentamt
European Patent Office
Office européen des brevets

(11) Publication number: 0 071 892 B1

(12) # EUROPEAN PATENT SPECIFICATION

(45) Date of publication of patent specification: 23.10.85

(51) Int. Cl.[4]: C 07 F 9/65, A 61 K 31/675

(21) Application number: 82106858.2

(22) Date of filing: 29.07.82

(54) Phospholipid derivatives, process for preparation thereof and pharmaceutical composition of the same.

(30) Priority: 31.07.81 GB 8123587
22.09.81 GB 8128648
28.04.82 GB 8212259

(43) Date of publication of application:
16.02.83 Bulletin 83/07

(45) Publication of the grant of the patent:
23.10.85 Bulletin 85/43

(84) Designated Contracting States:
BE CH DE FR GB IT LI LU NL SE

(56) References cited:
EP-A-0 035 375
DE-A-2 811 667

(73) Proprietor: Fujisawa Pharmaceutical Co., Ltd.
3, Doshomachi 4-chome Higashi-ku
Osaka-shi, Osaka 541 (JP)

(72) Inventor: Teraji, Tsutomu
No. 20-6, Kofudai 6-chome Toyono-cho
Toyono-gun Osaka 563-01 (JP)
Inventor: Todo, Eishiro
No. 2-2-304, Kitamidorigaoka 1-chome
Toyonaka-shi Osaka 560 (JP)
Inventor: Shimazaki, Norihiko
No. 45-23, Tarumi-cho 1-chome
Suita-shi Osaka 563 (JP)
Inventor: Oku, Teruo
No. 2-23-406, Wakayamadai 2-chome
Shimamoto-cho
Mishima-gun Osaka 618 (JP)
Inventor: Namiki, Takayuki
No. 8-22, Wakabaso-1 Sakurai 3-chome
Minoo-shi Osaka 562 (JP)

(74) Representative: Türk, Dietmar, Dr. rer. nat. et al
Redies, Redies, Türk & Gille Patentanwälte
Brucknerstrasse 20
D-4000 Düsseldorf 13 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 071 892 B1

Courier Press, Leamington Spa, England.

**Description**

This invention relates to phospholipid derivatives. More particularly, it relates to new phospholipid derivatives which have antitumor activity, to processes for the preparation thereof, and to pharmaceutical composition comprising the same for therapeutical treatment of cancer in human beings.

Accordingly, one object of this invention is to provide new and useful phospholipid derivatives.

Another object of this invention is to provide processes for preparation of phospholipid derivatives.

A further object of this invention is to provide useful pharmaceutical composition comprising said phospholipid derivatives as an antitumor agent.

Still further object of the present invention is to provide new phospholipid derivatives for use as an active substance for treating cancer.

The object phospholipid derivatives of the present invention are novel and include the compound of the formula (I):

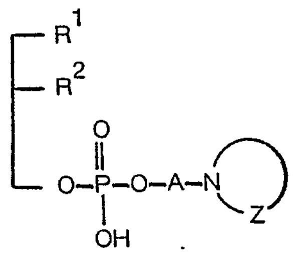

(I)

wherein

$R^1$ is $C_1$—$C_{25}$-alkoxy or -alkenyloxy;

$R^2$ is hydrogen, halogen, hydroxy, lower alkoxy, ar(lower)alkoxy, aryloxy, lower alkylthio, arylthio, thiadiazolylthio, lower alkylsulfonyl, lower alkoxycarbonylamino or lower alkylureido;

A is lower alkylene optionally interrupted by a —NHCO— group; and

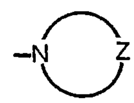

is unsaturated or saturated 5—7 membered heterocyclic group which may contain additional N, O or S atom(s), and may have 1 to 3 substituent(s) selected from lower alkyl, lower alkylamino, lower alkoxy, halogen, hydroxy, carboxy, lower alkoxycarbonyl and hydroxy(lower)alkyl groups;

provided that $R^1$ is alkoxy having 15 or more carbon atoms when

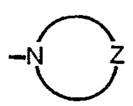

is a pyridinio group,

and pharmaceutically acceptable salts thereof.

In the above and subsequent description of the present specification, suitable examples and illustrations for the various definitions to be included within the scope of the invention are explained in details as follows.

The term "lower" is intended to mean 1 to 6 carbon atom(s) and the term "higher" is intended to mean 7 to 25 carbon atoms, unless otherwise indicated.

Suitable "alkoxy" for $R^1$ is straight or branched one containing 1 to 25 carbon atoms and may include methoxy, ethoxy, propoxy, isopropoxy, butoxy, tert-butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy, eicosyloxy, heneicosyloxy, docosyloxy, tricosyloxy, tetracosyloxy and pentacosyloxy, among which the preferable alkoxy for $R^1$ is the higher one having 15 or more carbon atoms.

Suitable "alkenyloxy" for $R^1$ is the one containing at least one double bond in the above exemplified "alkoxy" group for $R^1$. More particularly, vinyloxy, allyloxy, 1-propenyloxy, isopropenyloxy, 1-methylallyloxy, 1, 2 or 3-butenyloxy, 1, 2, 3 or 4-pentenyloxy, 1, 2, 3, 4 or 5-hexenyloxy, heptenyloxy, octenyloxy, nonenyloxy, decenyloxy, undecenyloxy, dodecenyloxy, tridecenyloxy, tetradecenyloxy, pentadecenyloxy, hexadecenyloxy, heptadecenyloxy, octadecenyloxy, nonadecenyloxy and eicosenyloxy are exemplified as suitable alkenyloxy for $R^1$. More preferable "alkenyloxy" is 8-hexadecenyloxy, 9-octadecenyloxy or 9,12-octadecadienyloxy.

"Halogen" for $R^2$ includes chlorine, bromine, iodine and fluorine.

Suitable "lower alkoxy" for $R^2$ and "lower alkoxy" moiety in the "ar(lower)alkoxy" and "lower alkoxycarbonylamino" for $R^2$ are the ones containing 1 to 6 carbon atoms among the aforementioned alkoxy.

Suitable "aryl" moiety in the "ar(lower)alkoxy", "aryloxy" and "arylthio" for $R^2$ may include phenyl, tolyl, xylyl, mesityl, cumenyl and naphthyl.

Suitable "lower alkyl" moiety in the "lower alkylthio", "lower alkylsulfonyl" and "lower alkylureido" for $R^2$ may include straight or branched ones containing 1 to 6 carbon atoms such as methyl, ethyl, propyl, isopropyl, butyl, isobutyl, tert-butyl, pentyl and hexyl.

Suitable "thiadiazolylthio" for $R^2$ may include 1,3,4-thiadiazol-5-ylthio, 1,3,4-thiadiazol-4-ylthio, 1,2,4-thiadiazol-5-ylthio, 1,2,4-thiadiazol-4-ylthio.

Suitable "lower alkylene" for A is straight or branched one containing 2 to 6 carbon atoms and may include ethylene, trimethylene, propylene, tetramethylene, pentamethylene and hexamethylene.

These "lower alkylene" may optionally be interrupted by a —NHCO— group at any portion therein.

Suitable "unsaturated or saturated 5—7 membered heterocyclic group which may contain additional N, O or S atom(s)" for

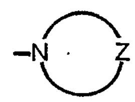

may include 1-pyrrolyl, 1-imidazolyl, 3-imidazolio, 1-pyrazolyl, 2-pyrazolio, 1-pyrrolidinyl, 1-pyrrolinyl, 1-imidazolidinyl, 2-imidazolin-1-yl, 1-pyrazolidinyl, 3-pyrazolin-1-yl, 1,3,4-triazol-1-yl, 1,2,4-triazol-1-yl, pyridinio, 1-pyrazinio, pyrimidinio, 1-pyridazinio, 1,2,4-triazin-1-io, piperidino, 1-piperazinyl, 3-oxazolio, 2-isoxazolio, morpholino, 3-thiazolio, 2-isothiazolio, 1,4-thiazin-4-io, 5,6-dihydro-1,4-thiazin-4-yl, 1-homopiperazinyl, homopiperidino and homomorpholino.

The heterocyclic ring as mentioned above may have 1 to 3 substituent(s) selected from the aforementioned lower alkyl, lower alkylamino (e.g. methylamino, ethylamino, propylamino, isopropylamino, butylamino, pentylamino, hexylamino, N,N-dimethylamino, N,N-diethylamino, N,N-dipropylamino), aforementioned lower alkoxy, aforementioned halogen, hydroxy, carboxy, lower alkoxycarbonyl (e.g. methoxycarbonyl, ethoxycarbonyl, propoxycarbonyl) and hydroxy(lower)alkyl (e.g. hydroxymethyl, hydroxyethyl, hydroxypropyl).

It is to be noted that the pharmaceutically acceptable salt of the object compound (I) includes an intramolecular salt as illustrated by the formula (I') and intermolecular salts as illustrated by formula (I''), (I''') and (I'''').

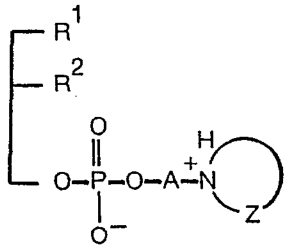
(I')

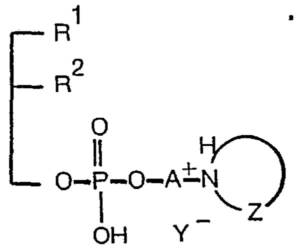
(I'')

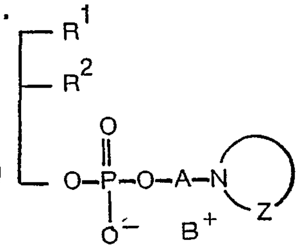
(I''')

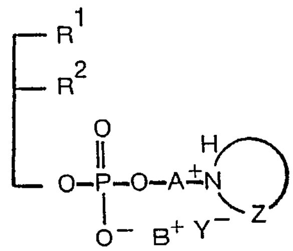
(I'''')

(in which $R^1$, $R^2$, A,

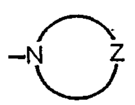

are each as defined above, $Y^-$ is an anion derived from an acid, and $B^+$ is a cation derived from a base).

The salt of type (I') may be a betain-type salt such as a pyridinio- or a piperazinio-phosphate. The salts of type (I''), (I''') and (I'''') may include intermolecular onium salts such as an acid addition salt (e.g. a pyridinium- or a piperazinium-salt), and a salt of the hydrogen phosphate with a base (e.g. a metal salt).

Suitable anions derived from acids for $Y^-$ includes an inorganic and organic ones such as a halide anion (e.g. chloride-, bromide-, or iodide-anion), a hydroxide anion, a sulfate anion, a sulfonate anion (e.g. methanesulfonyloxy-, benzenesulfonyloxy-, tosyloxy-, or camphorsulfonyloxy-anion), a carboxylate anion (e.g. trifluoroacetoxy- or hydrogen tartaroyloxy-anion). Suitable cations derived from bases for $B^+$ includes an alkali metal ion (e.g. sodium- or potassium-ion). The pharmaceutically acceptable salts of the compounds (I) also includes hydrates and solvates thereof.

With regard to the compounds (I), it is to be noted that the compounds [I] include all of the possible optical isomers due to the asymmetric carbon atom in the molecule of the compounds [I].

And further, it is to be noted that all of the chemical formulas of the 1-propanol moiety are shown by formula (A) in this specification instead of formula (B) for abbreviation sake.

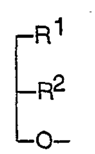
(A)

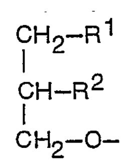
(B)

The compound (I) and its salt of the present invention can be prepared by the following processes.

PROCESS 1

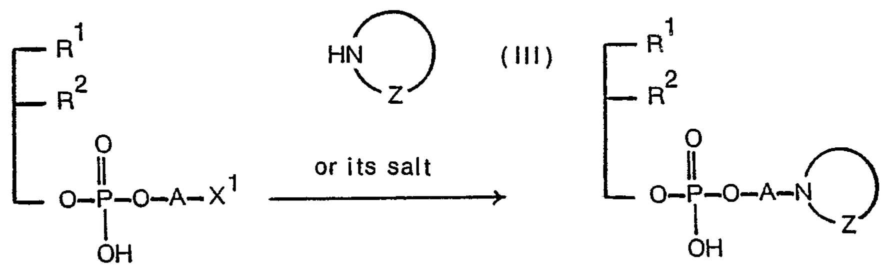


(II) or its salt

(I) or its salt

PROCESS 2

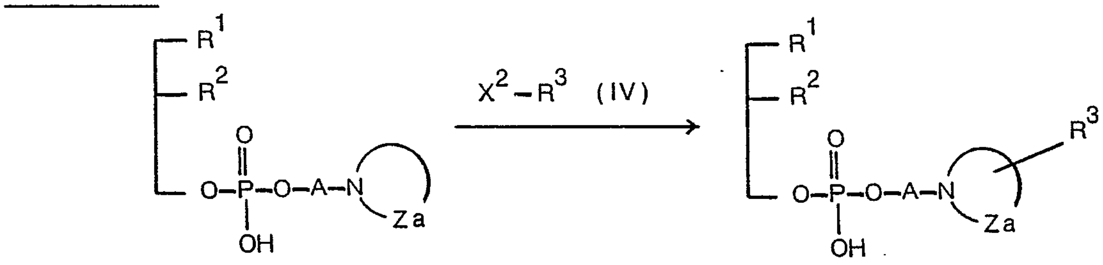


(Ib) or its salt

(Ia) or its salt

PROCESS 3

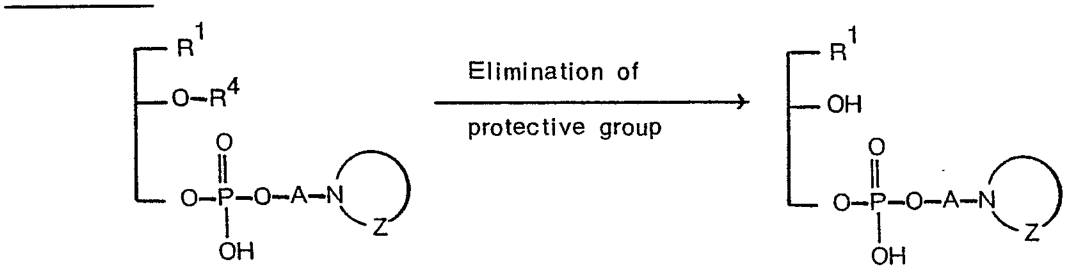


(V) or its salt

(Ic) or its salt

PROCESS 4

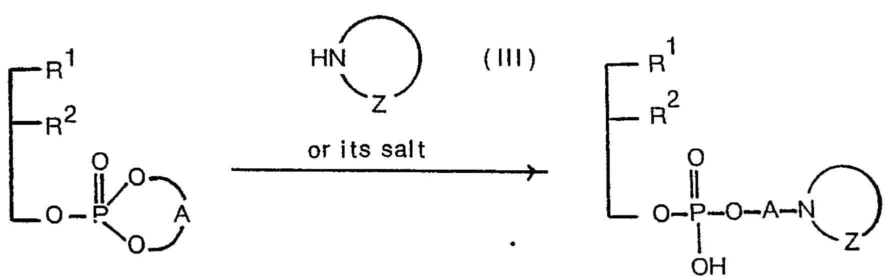


(VI) or its salt

(I) or its salt

wherein

$R^1$, $R^2$, A and 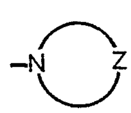 are each as defined above,

$X^1$ and $X^2$ are each an acid residue,

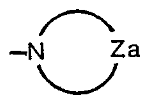

is unsaturated or saturated 5—7 membered heterocyclic group which may contain additional N, O or S atom(s), and may have 1 to 2 substituent(s) selected from lower alkyl, lower alkylamino, lower alkoxy, halogen, hydroxy, carboxy, lower alkoxycarbonyl and hydroxy(lower)alkyl groups,

$R^3$ is lower alkyl, and

$R^4$ is a protective group for hydroxy.

Suitable "acid residue" for $X^1$ and $X^2$ may include halogen (e.g. fluorine, chlorine, bromine, iodine), acyloxy (e.g. benzenesulfonyloxy, tosyloxy).

Suitable "unsaturated or saturated 5—7 membered heterocyclic group which may contain additional N, O or S atom(s), and may have 1 to 2 substituent(s) selected from lower alkyl, lower alkylamino, lower alkoxy, halogen, hydroxy, carboxy, lower alkoxycarbonyl and hydroxy(lower)alkyl group" for

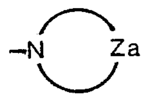

is the same as the one for

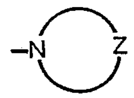

except the number of the substituents.

Suitable "lower alkyl" for $R^3$ is the same as mentioned before.

Suitable "protective groups for hydroxy" for $R^4$ are conventional protective groups such as lower alkoxycarbonyl (e.g. methoxycarbonyl, ethoxycarbonyl, propoxycarbonyl), benzyloxycarbonyl, alkanoyl (e.g. formyl, acetyl) and ar(lower)alkyl (e.g. benzyl, trityl).

The processes for preparing the compounds (I) and salts thereof of the present invention are explained in detail in the following.

*Process 1*

The compound (I) and its salt can be prepared by reacting a compound (II) or its salt with a compound (III) or its salt.

The suitable salts of the compound (II) are the metal salts as exemplified before for the compound (I).

The suitable salts of the compound (III) are the inorganic or organic acid salts as exemplified before for the compound (I).

This reaction is usually carried out in a solvent such as acetone, methanol, tetrahydrofuran, chloroform, benzene or any other solvent which does not adversely affect the reaction. When the compound (III) or its salt is liquid, the compound (III) or its salt can be used as a solvent, too.

The reaction temperature is not critical and the reaction is preferably carried out at ambient temperature or under heating.

When the compound (I) has a quartery nitrogen atom in the group of

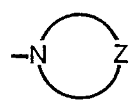

(e.g. pyridinio) and it is obtained in an acid addition salt form in the present reaction, the resulting acid addition salt can be optionally converted to its intramolecular salt by a conventional method, for instance, by treating the acid addition salt with an ion-exchange resin or silver acetate.

*Process 2*

The compound (Ia) and its salt can be prepared by reacting a compound (Ib) or its salt with a compound (IV).

The suitable salts of the compounds (Ia) and (Ib) can be referred to those as exemplified before for the compound (I).

This reaction is preferably carried out in the presence of an organic or inorganic base, for example, cyclohexylamine, di- or tri-(lower)alkylamine (e.g. trimethylamine, triethylamine, dimethylamine), pyridine, metal hydroxide (e.g. sodium hydroxide) or alkali metal hydride (e.g. sodium hydride, potassium hydride).

The reaction is usually carried out in a solvent such as methanol, ethanol, acetone, benzene or any other solvent which does not adversely affect the reaction.

The reaction temperature is not critical and the reaction is preferably carried out at ambient temperature or under warming or heating.

When the compound (Ia) is obtained in an acid addition salt form in the present reaction as mentioned before, the resulting acid addition salt can be optionally converted to its intramolecular salt according to the procedures as described in the above Process 1.

*Process 3*

The compound (Ic) and its salt can be prepared by subjecting a compound (V) or its salt to the elimination reaction of the protective group on the hydroxy group.

The suitable salts of the compound (V) and (Ic) can be referred to those as exemplified before for the compound (I).

The elimination reaction is carried out by a conventional method such as solvolysis (e.g. hydrolysis, alcoholysis), reduction or the like in accordance with a kind of the protective group.

Among these methods, when the protective group is a lower alkoxycarbonyl, benzyloxycarbonyl or ar(lower)alkanoyl, solvolysis is a preferable method. More specifically, hydrolysis by using a base or an acid is the most preferable.

Suitable base may include an organic or inorganic base such as alkali metal hydroxide (e.g. sodium hydroxide, potassium hydroxide), alkaline earth metal hydroxide (e.g. magnesium hydroxide), alkali metal carbonate or bicarbonate (e.g. sodium carbonate, sodium bicarbonate), alkali metal alkoxide (e.g. sodium methoxide, potassium methoxide) and organic amine (e.g. methylamine, diethylamine, pyrrolidine).

Suitable acid may include an inorganic or organic acid such as hydrochloric acid, hydrobromic acid, sulfuric acid and trifluoroacetic acid.

The reaction is usually carried out in a solvent such as water, methanol, ethanol, propanol, dimethylformamide or any other solvent which does not adversely affect the reaction, or a mixture thereof.

The reaction temperature is not critical, but the reaction is preferably carried out under mild condition such as under cooling at ambient temperature or under warming in order to avoid the hydrolysis of phosphate.

*Process 4*

The compound (I) and its salt can be prepared by reacting a compound (VI) or its salt with a compound (III) or its salt.

The suitable salts of the compound (VI) are the salts with an acid as exemplified before for the compound (I).

The reaction can be carried out by substantially the same manner as that of Process 1.

The desired compound prepared by the above-mentioned processes can be isolated from the reaction mixture and purified by a conventional method.

The compound (I) in a free form can optionally be converted to its salt, and the compound in an intramolecular salt form can also be converted to its acid addition salt by conventional manner.

Some of the starting compounds (II), (V) and (VI) are novel, and can be prepared by process as shown in the following Examples or processes chemically equivalent thereto.

The following pharmacological test data show that the object compounds (I) of the present invention exhibit high anti-tumor activity.

*Test Method A*

Groups of six female BALB/c mice, aged 12 weeks and weighing 19—21 g were used.

Fibrosarcoma Meth A (hereinafter referred to as Meth A) was successively transferred every 7 days into BALB/c mice by intraperitoneal inoculation of the ascites cells thereof and the Meth A in the ascites cells as harvested 6 days after the inoculation was used as tumor cells.

Each of the BALB/c mice was inoculated intradermally with $1 \times 10^5$ Meth A cells in 0.05 ml Hanks solution.

Test compound was dissolved in 0.5% methylcellulose saline solution, and was intratumorally injected to each of the mice in doses of 100 μg/mouse once daily from Day 1 to Day 4 postimplant.

The control group was given with a vehicle alone in the same way.

The antitumor effectiveness of the test compound was estimated by tumor growth inhibition (1—T/C).

T: Mean diameter of the growing tumor of the medicated group

C: Mean diameter of the growing tumor of the control group

Results of Test Method A

| Compounds Example No. | *a | | *b | | *c | |
|---|---|---|---|---|---|---|
| | Day 14 | Day 21 | Day 14 | Day 21 | Day 14 | Day 21 |
| 1 | 0 | 0 | 100 | 100 | 6/6 | 6/6 |
| 3—(1) | 0 | 0 | 100 | 100 | 6/6 | 6/6 |
| 3—(13) | 7.9 | 12.5 | 37 | 31 | 0/6 | 0/6 |
| 3—(21) | 0 | 0 | 100 | 100 | 6/6 | 6/6 |
| 3—(22) | 2.9 | 5.6 | 77 | 68 | 3/6 | 3/6 |
| 3—(23) | 2.8 | 5.3 | 77 | 70 | 3/6 | 3/6 |
| 3—(25) | 0.8 | 1.3 | 94 | 93 | 5/6 | 5/6 |
| 3—(30) | 1.6 | 2.8 | 85 | 84 | 4/6 | 4/6 |
| 9—(4) | 0.8 | 2.3 | 92 | 86 | 4/6 | 4/6 |

*a: Mean of two diameters of the growing tumor (mm)

*b: Tumor growth inhibition (%) [(1−T/c)×100]

*c: Ratio of tumor free mice [Number of tumor free mice/Number of mice used as a group]

*Test Method B*

Groups of eight female BALB/c mice, aged 8—9 weeks and weighing 18.0—22.5 g were used.

Fibrosarcoma Meth A (hereinafter referred to as Meth A) was successively transferred every 7 days into BALB/c mice by intraperitoneal inoculation of the ascites cells thereof and the Meth A in the ascites cells as harvested 6 or 7 days after the inoculation was used as tumor cells.

Each of the BALB/c mice was inoculated intrapleurally with 5 × $10^5$ Meth A cells in 0.1 ml Hanks solution.

Test compound was dissolved in phosphate buffer saline solution, and was injected into pleural cavity to each of the mice in doses of 100 μg/0.05 ml/mouse three times, i.e. before 14 days, after 1 hour and after 3 days of tumor implantation.

The control group was given with a vehicle alone in the same way.

The antitumor activity of the test compound was estimated by comparing mean survival time of the two groups.

T: Mean survival time of the medicated group

C: Mean survival time of the control group

Results of Test Method B

| Compound Example No. | Mean survival time (Day) | Anti-tumor activity (%) *d |
|---|---|---|
| 1 | 22.0 | 367 |
| 3–(1) | 20.5 | 342 |
| 3–(2) | 21.0 | 350 |
| 3–(3) | 19.0 | 317 |
| 3–(7) | 19.5 | 325 |
| 3–(9) | 21.0 | 350 |
| 3–(10) | 25.5 | 364 |
| 3–(12) | 20.0 | 333 |
| 3–(14) | 19.5 | 325 |
| 3–(15) | 26.0 | 433 |
| 3–(16) | 21.0 | 350 |
| 3–(18) | 19.5 | 325 |
| 3–(19) | 19.0 | 317 |
| 3–(26) | >39.0 | >650 |
| 3–(29) | 18.5 | 264 |
| 3–(31) | 21.5 | 358 |
| 5–(2) | 25.5 | 364 |
| 8–(4) | 25.0 | 417 |
| 10–(6) | 24.0 | <400 |
| 11–(2) | 21.0 | 350 |
| *e | 14.5 | 242 |

*d: T/C × 100

*e: (rac)-1-O-Octadecyl-2-O-methyl-glycerol-3-phosphorylcholine which is described in British Patent No. 1583661.

As being apparent from the above test results, the compounds (I) of the present invention are useful as antitumor agents.

The effective ingredient may usually be administered with a dose of 0.1 mg/kg to 500 mg/kg, 1 to 4 times a day in a preparations such as tablet, granule, powder, capsule, syrup, injection and suppository. However, the above dosage may be increased or decreased according to the age, weight or conditions of the patient or the administering method.

The above mentioned pharmaceutical preparations can be prepared in a conventional manner by using conventional carriers and additives.

The present invention is illustrated by the following Examples in more detail.

Example 1

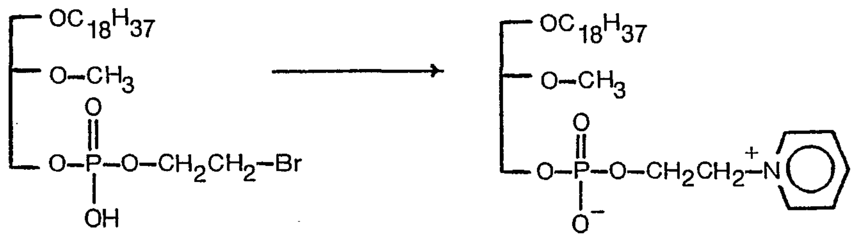

A mixture of (rac)-1-O-octadecyl-2-O-methyl-glycerol-3-(2-bromoethyl)phosphate (2.18 g) and pyridine (10 ml) was stirred at 100°C for one hour, and then the solvent was removed under reduced pressure. The residue was titurated with acetone. The powder was dissolved in a mixture of 90% aqeuous methanol (20 ml) and chloroform (4 ml), and the mixture was treated with silver acetate (1.36 g) for 30 minutes. The precipitate was removed by filtration and washed with methanol. The filtrate and washings were combined and evaporated under reduced pressure. The residue was purified by column chromatography on silica gel (66 g, elution by chloroform-methanol-water, 65:25:4) to give 1.15 g of (rac)-1-O-octadecyl-2-O-methyl-glycerol-3-[2-(1-pyridinio)ethyl]phosphate, which was recrystallized from chloroform-acetone, yield 0.99 g (colorless crystals). m.p. 84.5 to 89°C.

I.R. (Nujol®): 3380, 1590, 1220, 1080 $cm^{-1}$

N.M.R. ($CD_3OD$) ppm: 0.90—1.75 (35H, m), 3.42 (3H, s), 3.48—4.95 (11H, m), 8.10—9.00 (5H, m)

| | | | |
|---|---|---|---|
| Anal. Calcd. for $C_{29}H_{54}O_6NP{\cdot}H_2O$: | C: 62.01, | H: 10.04, | N, 2.49 |
| Found: | C: 61.32, | H: 9.99, | N: 2.51 |

Example 2

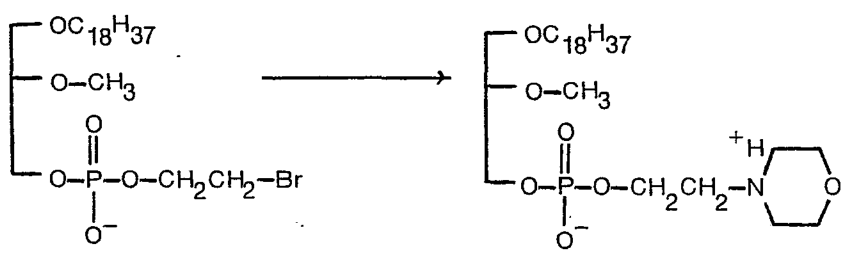

A mixture of (rac)-1-O-octadecyl-2-O-methyl-glycerol-3-(2-bromoethyl)phosphate (1.09 g) and morpholine (2 ml) was stirred for 4 hours at ambient temperature, and then the mixture was evaporated to dryness. The residue dissolved in a mixture of chloroform-methanol-water (1:2:1) was passed through a mixed ion-exchange column, containing Amberlite IRC—50 (Trade name, manufactured by Rohm & Haas) ($H^+$) (3 g) and Amberlite IR—45 (Trade name, manufactured by Rohm & Haas) ($OH^-$) (6 g) to give 1.25 g of (rac)-1-O-octadecyl-2-O-methyl-glycerol-3-(2-morpholinoethyl)phosphate, which was purified by column chromatography on silica gel (30 g, elution by chloroform-methanol, 9:1 ~ 9:2) and recrystallized from chloroform-acetone, yield 0.95 g (colorless solid). m.p. 45°C

I.R. (Nujol®): 3350, 1220, 1115 $cm^{-1}$

N.M.R. ($CD_3OD$) ppm: 0.7—1.9 (35H, m), 2.4—2.9 (6H, m), 3.2—4.2 (13H, m), 3.47 (3H, s)

Example 3

The following compounds were prepared according to the similar manners to those of Examples 1 and 2.

(1)

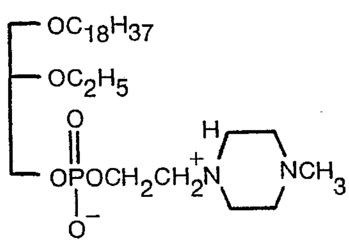

(rac)-1-O-Octadecyl-2-O-ethyl-glycerol-3-[2-(4-methyl-1-piperazinyl)ethyl]phosphate. m.p. 115°C

I.R. (Nujol®): 3400, 1645, 1215, 1090, 1055 $cm^{-1}$

N.M.R. ($CD_3OD$) ppm: 0.90 (3H, t, J = 5.5 Hz), 1.07—1.69 (35H, m), 2.71 (3H, s), 2.80—4.19 (20H, m), 4.74 (1H, m)

Anal. Calcd. for $C_{30}H_{63}N_2O_6P \cdot H_2O$: C: 60.37, H: 10.99, N: 4.69
Found: C: 60.15, H: 11.34, N: 4.50

(2)

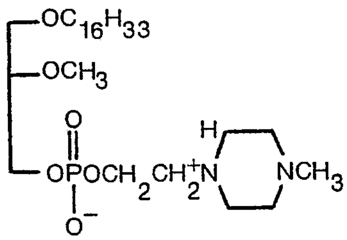

(rac)-1-O-Hexadecyl-2-O-methyl-glycerol-3-[2-(4-methyl-1-piperazinyl)ethyl]phosphate. m.p. 125 to 128°C

I.R. (Nujol®): 3350, 1220 $cm^{-1}$

N.M.R. ($CD_3OD$) ppm: 0.9 (3H, m), 1.0—1.8 (28H, m), 2.68 (3H, s), 2.76—3.16 (9H, m), 3.3—4.2 (10H, m), 3.46 (3H, s)

Anal. Calcd. for $C_{27}H_{57}N_2O_6P \cdot H_2O$: C: 58.45, H: 10.71, N: 5.05
Found: C: 58.28, H: 11.07, N: 5.05

(3)

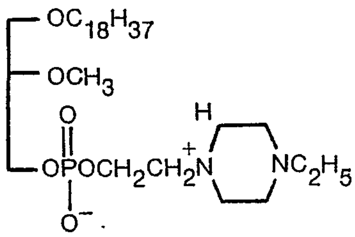

(rac)-1-O-octadecyl-2-O-methyl-glycerol-3-[2-(4-ethyl-1-piperazinyl)ethyl]phosphate. m.p. 150 to 155°C

I.R. (Nujol®): 3350, 1210, 1050 $cm^{-1}$

N.M.R. ($CD_3OD$) ppm: 0.89 (3H, t, J = 5.5Hz), 1.05—1.69 (35H, m), 2.79—3.14 (12H, m), 3.34—3.56 (4H, m), 3.44 (3H, s), 3.83—4.13 (4H, m), 4.68 (1H, m)

Anal. Calcd. for $C_{30}H_{63}N_2O_6P . H_2O$: C: 60.37, H: 10.98, N: 4.69
Found: C: 60.68, H: 11.08, N: 4.62

(4)

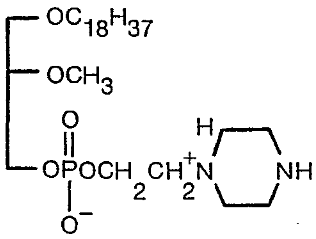

(rac)-1-O-Oexadecyl-2-O-methyl-glycerol-3-[2-(1-piperazinyl)ethyl]phosphate. m.p. 208°C

I.R. (Nujol®): 3350, 1625, 1220, 1070 $cm^{-1}$

N.M.R. ($CD_3OD$) ppm: 0.90 (3H, t, J = 5.5Hz), 1.05—1.68 (32H, m), 2.58—2.89 (6H, m), 3.00—3.34 (6H, m), 3.47 (3H, s), 3.36—4.09 (6H, m), 4.74 (1H, m)

Anal. Calcd. for $C_{28}H_{59}N_2O_6P . H_2O$: C: 59.13, H: 10.81, N: 4.93
Found: C: 59.42, H: 10.82, N: 4.89

(5)

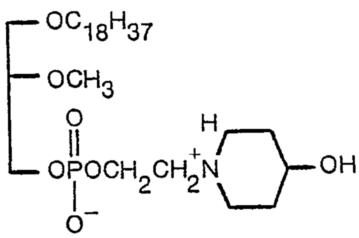

(rac)-1-O-Octadecyl-2-O-methyl-glycerol-3-[2-(4-hydroxypiperadino)ethyl]phosphate. m.p. 210°C

I.R. (Nujol®): 3200, 1215, 1055 $cm^{-1}$

N.M.R. ($CD_3OD$) ppm: 0.90 (3H, t, J = 5.5Hz), 1.07—1.66 (32H, m), 1.69—2.25 (4H, m), 3.13—3.68 (10H, m), 3.46 (3H, s), 3.79—4.29 (5H, m), 4.71 (1H, m)

| | | | |
|---|---|---|---|
| Anal. Calcd. for $C_{29}H_{60}NO_7P$ . $1/2H_2O$: | C: 60.60, | H: 10.70, | N: 2.44 |
| Found: | C: 60.75, | H: 11.11, | N: 2.43 |

(6)

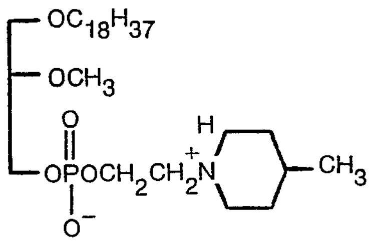

(rac)-1-O-Octadecyl-2-O-methyl-glycerol-3-[2-(4-methylpiperadino)ethyl]phosphate. m.p. 97 to 100°C

I.R. (Nujol®): 3400, 1220, 1040 $cm^{-1}$

N.M.R. ($CD_3OD$) ppm: 0.90 (3H, t, J = 5.5Hz), 1.04 (3H, d, J = 5Hz), 1.09—1.49 (32H, m), 1.50—2.01 (4H, m), 2.85—3.69 (11H, m), 3.46 (3H, s), 3.82—4.01 (2H, m), 4.06—4.29 (2H, m), 4.71 (1H, m)

| | | | |
|---|---|---|---|
| Anal. Calcd. for $C_{30}H_{62}NO_6P$ . $1/2H_2O$: | C: 62.91, | H: 11.09, | N: 2.45 |
| Found: | C: 62.79, | H: 11.05, | N: 2.45 |

(7)

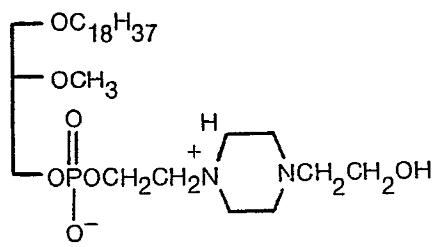

(rac)-1-O-Octadecyl-2-O-methyl-glycerol-3-[2-[4-(2-hydroxyethyl)-1-piperazinyl]ethyl]phosphate. m.p. 196 to 199°C

I.R. (Nujol®): 3120, 2200, 1210, 1050 $cm^{-1}$

N.M.R. ($CD_3OD$) ppm: 0.90 (3H, t, J = 5.5Hz), 1.07—1.81 (32H, m), 2.78—3.22 (10H, m), 3.26—3.65 (6H, m), 3.46 (3H, s), 3.69—3.85 (2H, m), 3.85—3.99 (2H, m), 4.00—4.21 (2H, m), 4.75 (1H, m)

| | | | |
|---|---|---|---|
| Anal. Calcd. for $C_{30}H_{63}N_2O_7P$ . $1/2H_2O$: | C: 59.60, | H: 10.69, | N: 4.52 |
| Found: | C: 59.82, | H: 10.71, | N: 4.65 |

(8)

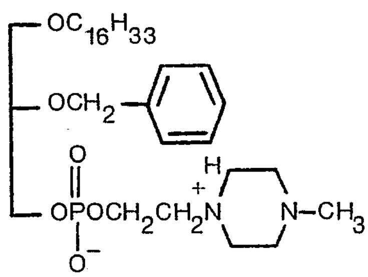

(rac)-1-O-Hexadecyl-2-O-benzyl-glycerol-3-[2-(4-methyl-1-piperazinyl)ethyl]phosphate. waxy solid.

I.R. (Film): 3350, 2700, 1640, 1200, 1050 $cm^{-1}$

N.M.R. ($CD_3OD$) ppm: 0.88 (3H, t, J = 5.5Hz), 1.08—1.83 (28H, m), 2.62 (3H, s), 2.68—3.13 (8H, m), 3.30—3.63 (4H, m), 3.66—4.20 (6H, m), 4.67 (2H, s), 4.70—4.98 (1H, m), 7.17—7.50 (5H, m)

| | | | |
|---|---|---|---|
| Anal. Calcd. for $C_{33}H_{61}N_2O_6P$ . $H_2O$: | C: 62.83, | H: 10.06, | N: 4.44 |
| Found: | C: 62.86, | H: 9.57, | N: 4.46 |

(9)

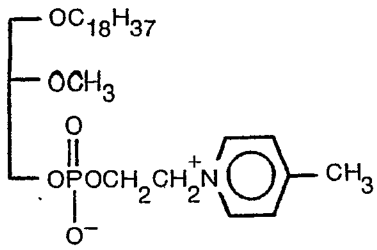

(rac)-1-O-Octadecyl-2-O-methyl-glycerol-3-[2-(4-methyl-1-pyridinio)ethyl]phosphate. m.p. 134°C.

I.R. (Nujol®): 3350, 1640, 1240 $cm^{-1}$

N.M.R. ($CD_3OD$) ppm: 0.86—1.66 (35H, m), 2.68 (3H, s), 3.42 (3H s), 3.20—4.86 (11H, m), 7.96 (2H, d, J = 7.5Hz), 8.84 (2H, d, J = 7.5Hz)

| | | | |
|---|---|---|---|
| Anal. Calcd. for $C_{30}H_{56}NO_6P \cdot H_2O$: | C: 61.66, | H: 10.16, | N: 2.39 |
| Found: | C: 61.45, | H: 10.70, | N: 2.31 |

(10)

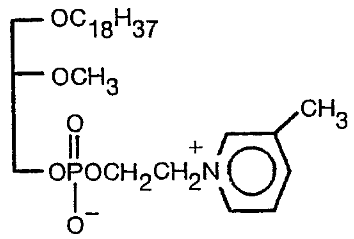

(rac)-1-O-Octadecyl-2-O-methyl-glycerol-3-[2-(3-methyl-1-piperidinio)ethyl]phosphate. m.p. 170°C.

I.R. ($CHCl_3$): 3650, 3300, 2850, 1635 $cm^{-1}$

N.M.R. ($CD_3OD$) ppm: 0.89 (3H, t, J = 6Hz), 1.06—1.70 (32H, m), 2.60 (3H, s), 3.44 (3H, s), 3.28—4.93 (11H, m), 8.03 (1H, dd, J = 6.5Hz, 7Hz) 8.87 (1H, d, J = 6.5Hz), 8.92 (1H, s)

| | | | |
|---|---|---|---|
| Anal. Calcd. for $C_{30}H_{56}NO_6P \cdot 3/2H_2O$: | C: 59.78, | H: 10.20, | N: 2.32 |
| Found: | C: 59.69, | H: 10.04, | N: 2.26 |

(11)

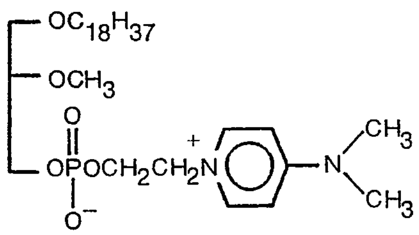

(rac)-1-O-Octadecyl-2-O-methyl-glycerol-3-[2-(4-dimethylamino-1-piperidinio)ethyl]phosphate. m.p. 208°C (dec.).

I.R. (Nujol®): 3350, 1650, 1570, 1230 $cm^{-1}$

N.M.R. ($CD_3OD$) ppm: 0.90—1.72 (35H, m), 3.27 (6H, s), 3.45 (3H, s), 3.12—4.56 (11H, m), 7.06 (2H, d, J = 8Hz), 8.27 (2H, d, J = 8Hz)

| | | | |
|---|---|---|---|
| Anal. Calcd. for $C_{31}H_{59}N_2O_6P \cdot 2H_2O$: | C: 58.93, | H: 10.05, | N: 4.43 |
| Found: | C: 59.16, | H: 10.42, | N: 3.97 |

(12)

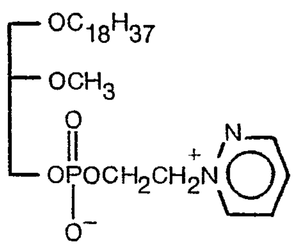

(rac)-1-O-Octadecyl-2-O-methyl-glycerol-3-[2-(1-pyridazinio)ethyl]phosphate. m.p. 74°C.

I.R. (Nujol®): 3350, 1600, 1245 $cm^{-1}$

N.M.R. ($CD_3OD$) ppm: 0.88—1.76 (35H, m), 3.36—5.06 (11H, m), 3.42 (3H, s), 8.34 (2H, m), 9.48 (1H, m), 9.76 (1H, m)

Anal. Calcd. for $C_{28}H_{53}N_2O_6P \cdot H_2O$: C: 59.76, H: 9.85, N: 4.98
Found: C: 59.24, H: 9.96, N: 4.83

(13)

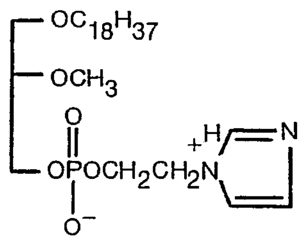

(rac)-1-O-Octadecyl-2-O-methyl-glycerol-3-[2-(1-imidazolyl)ethyl]phosphate. m.p. 238°C (dec.).

I.R. (Nujol®): 3100, 1260, 1120, 1085, 1050, 930, 850, 770 $cm^{-1}$

N.M.R. ($CD_3OD$-$CDCl_3$) ppm: 0.5—2.2 (35H, m), 3.14—3.6 (5H, m), 3.40 (3H, s), 3.6—3.96 (2H, m), 3.96—4.3 (4H, m), 6.92 (1H, s), 7.08 (1H, s), 7.62 (1H, s)

Anal. Calcd. for $C_{27}H_{53}N_2O_6P \cdot H_2O$: C: 58.87, H: 10.24, N: 5.09
Found: C: 58.50, H: 9.67, N: 4.89

(14)

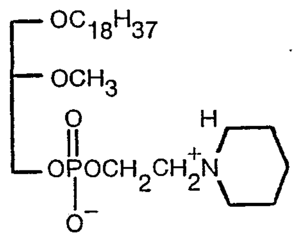

(rac)-1-O-Octadecyl-2-O-methyl-glycerol-3-[2-(piperidinoethyl]phosphate. m.p. 122 to 130°C.

I.R. (Nujol®): 3400, 1660, 1230, 1120, 1085, 1070, 1055, 1020 $cm^{-1}$

N.M.R. ($CD_3OD$) ppm: 0.6—2.0 (41H, m), 3.0—3.68 (11H, m) 3.44 (3H, s), 3.8—4.0 (2H, m), 4.04—4.3 (2H, m)

Anal. Calcd. for $C_{29}H_{60}NO_6P \cdot 2H_2O$: C: 59.46, H: 11.01, N: 2.39
Found: C: 59.30, H: 11.03, N: 2.28

(15)

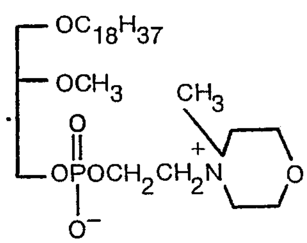

(rac)-1-O-Octadecyl-2-O-methyl-glycerol-3-[2-(N-methyl-4-morpholino)ethyl]phosphate. hygroscopic solid. m.p. 58°C.

I.R. (Nujol®): 3350, 1625, 1220 $cm^{-1}$

(16)

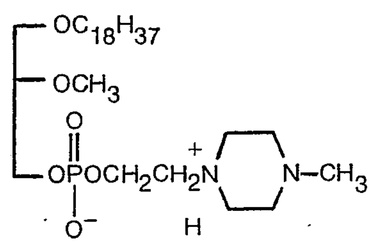

(rac)-1-O-Octadecyl-2-O-methyl-glycerol-3-[2-(4-methyl-1-piperazinyl)ethyl]phosphate. m.p. 132 to 136°C.

I.R. (Nujol®): 3350, 1650, 1210, 1050 $cm^{-1}$

N.M.R. ($CD_3OD$) ppm: 0.6—1.8 (35H, m), 2.68 (3H, s), 2.76—3.2 (10 H, m), 3.2—3.7 (5H, m), 3.46 (3H, s), 3.7—4.2 (4H, m)

Anal. Calcd. for $C_{29}H_{61}N_2O_6P \cdot 2H_2O$: C: 57.97, H: 10.91, N: 4.66
Found: C: 58.48, H: 11.23, N: 4.62

(17)

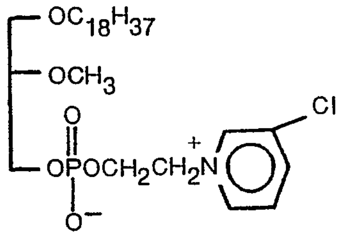

(rac)-1-O-Octadecyl-2-O-methyl-glycerol-3-[2-(3-chloro-1-piperadinio)ethyl]phosphate. m.p. 192°C.
I.R. (Nujol®): 3350, 1625, 1240 $cm^{-1}$
N.M.R. ($CD_3OD$) ppm: 0.92 (3H, t, J = 6Hz), 1.04—1.72 (32H, m), 3.46 (3H, s), 3.40—4.92 (11H, m), 8.18 (1H, dd, J = 6Hz, 7Hz), 8.74 (1H, d, J = 7Hz), 9.06 (1H, d, J = 6Hz), 9.36 (1H, s)

(18)

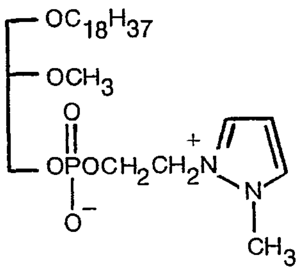

(rac)-1-O-Octadecyl-2-O-methyl-glycerol-3-[2-(4-methyl-2-pyrazolio)ethyl]phosphate. m.p. 218°C.
I.R. (Nujol®): 3370, 1675 (s), 1240 $cm^{-1}$
N.M.R. ($CD_3OD$) ppm: 0.90 (3H, t, J = 6Hz), 3.46 (3H, s), 4.25 (3H, s), 3.38—4.84 (11H, m), 6.84 (1H, dd, J = 3Hz, 4Hz), 8.38 (1H, d, J = 3Hz), 8.48 (1H, d, J = 4Hz)

| | | | |
|---|---|---|---|
| Anal. Calcd. for $C_{28}H_{55}N_2O_6P \cdot 3/2H_2O$: | C: 58.61, | H: 10.18, | N: 4.48 |
| Found: | C: 58.49, | H: 10.27, | N: 4.63 |

(19)

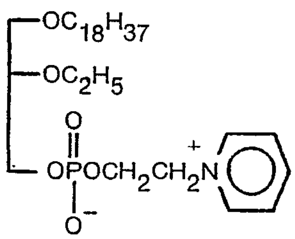

(rac)-1-O-Octadecyl-2-O-ethyl-glycerol-3-[2-(1-pyridinio)ethyl]phosphate. m.p. 190 to 195°C.
I.R. ($CHCl_3$) ppm: 3300, 1635, 1240 $cm^{-1}$
N.M.R. ($CD_3OD$) ppm: 0.89 (3H, t, J = 6.5Hz), 1.09—1.49 (35H, m), 3.28—4.97 (13H, m), 8.08—9.13 (5H, m)

| | | | |
|---|---|---|---|
| Anal. Calcd. for $C_{30}H_{56}NO_6P \cdot 1/2H_2O$: | C: 61.83, | H: 9.86, | N: 2.40 |
| Found: | C: 61.27, | H: 10.10, | N: 2.17 |

(20)

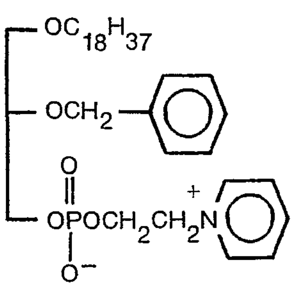

(rac)-1-O-Octadecyl-2-O-benzyl-glycerol-3-[2-(1-pyridinio)ethyl]phosphate. m.p. 113°C (dec.).
I.R. (Nujol.): 3370, 1230 $cm^{-1}$
N.M.R. ($CD_3OD$) ppm: 0.85 (3H, m), 1.0—2.0 (32H, m), 3.20—4.4 (11H, m), 4.68 (2H, s), 7.36 (5H, brs), 8.04—8.92 (5H, m)

| | | | |
|---|---|---|---|
| Anal. Calcd. for $C_{35}H_{58}NO_6P \cdot 2H_2O$: | C: 64.01, | H: 9.53, | N: 2.14 |
| Found: | C: 63.73, | H: 9.37, | N: 2.10 |

(21)

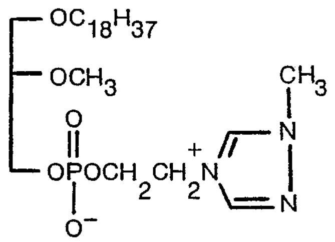

(rac)-1-O-Octadecyl-2-O-methyl-glycerol-3-[2-(1-methyl-1,2,4-triazol-4-io)ethyl]phosphate. m.p. 200°C (dec.).

I.R. ($CDCl_3$): 3280, 3130, 2900, 2850, 1580 $cm^{-1}$

N.M.R. ($CDCl_3$) ppm: 0.89 (3H, t, J = 5Hz), 1.12—1.87 (32H, m), 3.40 (3H, s), 4.18 (3H, s), 3.20—4.87 (11H, m), 9.05 (1H, s), 11.10 (1H, s)

| | | | |
|---|---|---|---|
| Anal. Calcd. for $C_{27}H_{54}N_3O_6P \cdot 1/2H_2O$: | C: 58.25, | H: 9.96, | N: 7.55 |
| Found: | C: 58.68, | H: 10.16, | N: 7.07 |

(22)

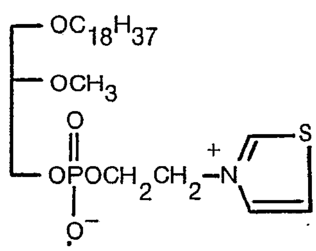

(rac)-1-O-Octadecyl-2-O-methyl-glycerol-3-[2-(3-thiazolio)ethyl]phosphate. m.p. 218 to 220°C.

I.R. ($CHCl_3$): 3300, 2900, 2850, 1660, 1460 $cm^{-1}$

N.M.R. ($CD_3OD$) ppm: 0.90 (3H, t, J = 5Hz), 1.09—1.58 (32H, m), 3.45 (3H, s), 3.23—4.97 (11H, m), 8.28 (1H, m), 8.51 (1H, m), 10.15 (1H, m)

| | | | |
|---|---|---|---|
| Anal. Calcd. for $C_{27}H_{52}NO_6PS \cdot 2H_2O$: | C: 55.36, | H: 9.63, | N: 2.39 |
| Found: | C: 55.47, | H: 9.29, | N: 2.33 |

(23)

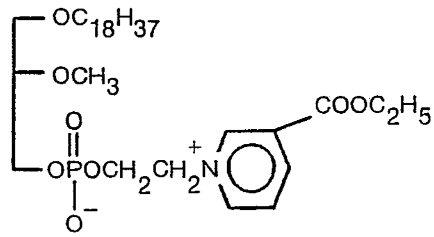

(rac)-1-O-Octadecyl-2-O-methyl-glycerol-3-[2-(3-ethoxycarbonyl-1-pyridinio)ethyl]phosphate. m.p. 140°C (dec.).

I.R. ($CHCl_3$): 3300, 2900, 2850, 1740, 1460 $cm^{-1}$

N.M.R. ($CD_3OD$) ppm: 0.91 (3H, t, J = 5.5Hz), 1.07—1.72 (32H, m), 1.48 (3H, t, J = 7Hz), 3.27—5.10 (11H, m), 4.58 (2H, q, J = 7Hz), 3.46 (3H, s), 8.34—9.67 (4H, m)

| | | | |
|---|---|---|---|
| Anal. Calcd. for $C_{23}H_{58}NO_8P \cdot 3/2H_2O$: | C: 59.79, | H: 9.56, | N: 2.18 |
| Found: | C: 60.07, | H: 9.59, | N: 2.00 |

(24)

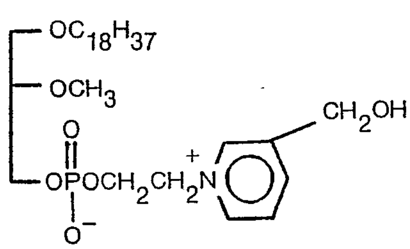

(rac)-1-O-Octadecyl-2-O-methyl-glycerol-3-[2-(3-hydroxymethyl-1-pyridinio)ethyl]phosphate. m.p. 160°C (dec.).

I.R. ($CHCl_3$): 3300, 2900, 2850, 1570, 1460 $cm^{-1}$

N.M.R. ($CD_3OD$) ppm: 0.91 (3H, t, J = 6Hz), 1.12—1.70 (32H, m), 3.28—4.98 (11H, m), 3.45 (3H, s), 4.89 (2H, s), 8.14 (1H, t, J = 8Hz), 8.62 (1H, d, J = 8Hz), 8.97 (1H, d, J = 8Hz), 9.02 (1H, s)

| | | | |
|---|---|---|---|
| Anal. Calcd. for $C_{30}H_{56}NO_7P$ . $3/2H_2O$: | C: 59.98, | H: 9.90, | N: 2.33 |
| Found: | C: 60.14, | H: 10.38, | N: 2.26 |

(25)

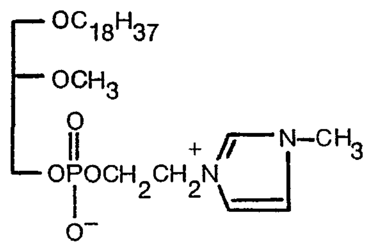

(rac)-1-O-Octadecyl-2-O-methyl-glycerol-3-[2-(1-methyl-3-imidazolio)ethyl]phosphate. m.p. 240 to 248°C.

I.R. ($CHCl_3$): 3300, 2900, 2850, 1570, 1460 $cm^{-1}$

N.M.R. ($CD_3OD$) ppm: 0.90 (3H, t, J = 6Hz), 1.12—1.67 (32H, m), 3.25—4.72 (11H, m), 3.45 (3H, s), 3.95 (3H, s), 7.57 (1H, m), 7.66 (1H, m), 8.95 (1H, s)

| | | | |
|---|---|---|---|
| Anal. Calcd. for $C_{28}H_{55}N_2O_6P$ . $H_2O$: | C: 59.55, | H: 10.17, | N: 4.96 |
| Found: | C: 59.37, | H: 10.69, | N: 4.75 |

(26)

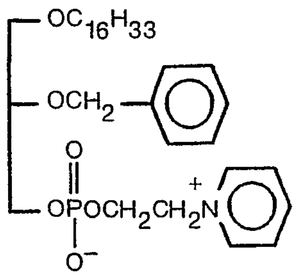

(rac)-1-O-Hexadecyl-2-O-benzyl-glycerol-3-[2-(1-pyridinio)ethyl]phosphate. m.p. 160°C (dec.).

I.R. (Nujol®): 3350, 3050, 1640, 1230, 1100, 1070 $cm^{-1}$

N.M.R. ($CD_3OD$) ppm: 0.7—1.0 (3H, m), 1.0—1.8 (28H, m), 3.2—4.46 (11H, m), 4.74 (2H, s), 7.20 (5H, s), 8.12—9.00 (5H, m)

| | | | |
|---|---|---|---|
| Anal. Calcd. for $C_{33}H_{54}NO_6P$ . $2H_2O$: | C: 63.13, | H: 9.31, | N: 2.23 |
| Found: | C: 63.91, | H: 9.18, | N: 2.19 |

(27)

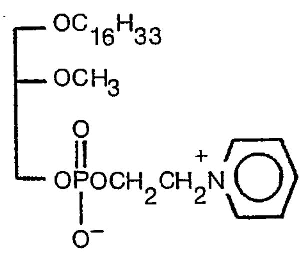

(rac)-1-O-Hexadecyl-2-O-methyl-glycerol-3-[2-(1-pyridinio)ethyl]phosphate. m.p. 157°C (dec.).

I.R. (Nujol®): 3370, 1640, 1245, 1130, 1080 $cm^{-1}$

N.M.R. ($CD_3OD$) ppm: 0.92 (3H, t, J = 5.5Hz), 1.04—1.80 (28H, m), 3.48 (3H, s), 3.30—4.96 (11H, m), 8.22—9.12 (5H, m)

| | | | |
|---|---|---|---|
| Anal. Calcd. for $C_{27}H_{50}NO_6P$ . $3/2H_2O$: | C: 59.76, | H: 9.84, | N: 2.58 |
| Found: | C: 59.78, | H: 9.97, | N: 2.62 |

(28)

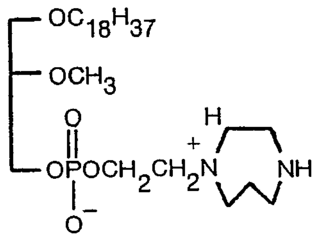

(rac)-1-O-Octadecyl-2-O-methyl-glycerol-3-[2-(1-homopiperazinyl)ethyl]phosphate. waxy solid.
I.R. (Nujol®): 3400, 1620, 1210, 1050 $cm^{-1}$

(29)

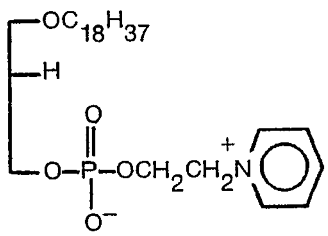

(rac)-1-O-Octadecyl-1,3-propanediol-3-[2-(1-pyridinio)ethyl]phosphate. m.p. 203°C (dec.).
I.R. (Nujol®): 3390, 1685, 1640, 1490 $cm^{-1}$
N.M.R. ($CD_3OD$) ppm: 0.89 (3H, t, J = 6Hz), 1.05—1.57 (32H, m), 1.78 (2H, m), 3.23—4.93 (10H, m), 7.94—9.03 (2H, m)

| | | | |
|---|---|---|---|
| Anal. Calcd. for $C_{28}H_{52}NO_5P$ . 3/2$H_2O$: | C: 62.19, | H: 10.25, | N: 2.59 |
| Found: | C: 62.27, | H: 10.49, | N: 2.53 |

(30)

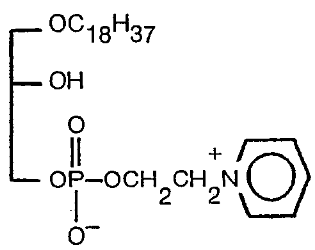

(rac)-1-O-Octadecyl-glycerol-3-[2-(1-pyridinio)ethyl]phosphate. m.p. 150°C (dec.).
I.R. (Nujol®): 3410, 1635, 1235 $cm^{-1}$

(31)

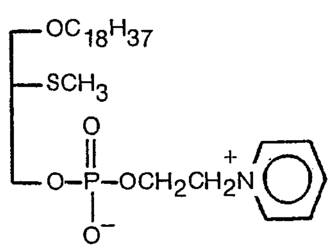

(rac)-1-O-Octadecyl-2-methylthio-1,3-propanediol-3-[2-(1-pyridinio)ethyl]phosphate. m.p. 194 to 195°C.
I.R. (Nujol®): 3350, 1635, 1240 $cm^{-1}$

(32)

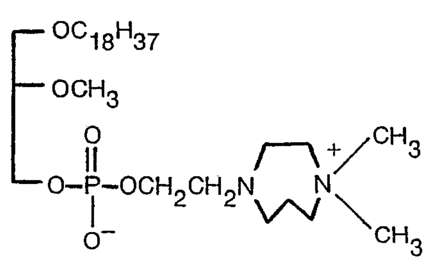

(rac)-1-O-Octadecyl-2-O-methyl-glycerol-3-[2-(4-dimethyl-1-homopiperazinio)ethyl]phosphate. m.p. 210 to 214°C.
I.R. (Nujol®): 3330, 1240, 1095 $cm^{-1}$

(33) 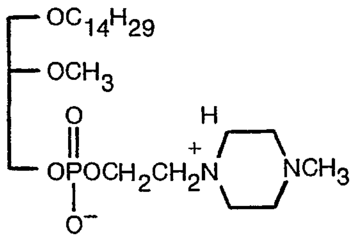

(rac)-1-O-Tetradecyl-2-O-methyl-glycerol-3-[2-(4-methyl-1-piperazinyl)ethyl]phosphate. waxy solid.

I.R. (Film): 3400, 2800, 1650, 1220, 1050 $cm^{-1}$

N.M.R. ($CD_3OD$) ppm: 0.89 (3H, t, J=5.5Hz), 1.10—1.82 (24H, m), 2.70 (3H, s), 2.77—4.27 (18H, m), 3.47 (3H, s), 4.67—4.98 (1H, m)

| | | | |
|---|---|---|---|
| Anal. Calcd. for $C_{25}H_{53}N_2O_6P.\frac{1}{2}H_2O$: | C: 58.00, | H: 10.51, | N: 5.41 |
| Found: | C: 57.82, | H: 10.31, | N: 5.39 |

(34) 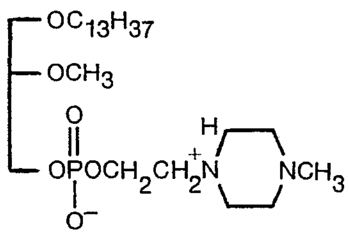

(rac)-1-O-Tridecyl-2-O-methyl-glycerol-3-[2-(4-methyl-1-piperazinyl)ethyl]phosphate.

N.M.R. ($CD_3OD$) ppm: 0.9 (3H, m), 1.0—1.7 (22H, m), 2.68 (3H, s), 2.76—3.16 (9H, m), 3.3—3.7 (6H, m), 3.46 (3H, s), 3.7—4.2 (4H, m)

Example 4

(1) To a mixture of (rac)-1-O-octadecyl-3-O-trityl-glycerol (11.74 g), triethylamine (3.03 g), and 4-dimethylaminopyridine (0.25 g) in dry 1,2-dichloroethane (110 ml) was added p-toluenesulfonyl chloride (5.70 g) in one portion at ambient temperature. After being stirred for 17 hours at the same temperature, the mixture was washed with water, an aqueous sodium bicarbonate solution, 5% aqueous sulfuric acid, and water respectively. The organic layer was dried and evaporated to dryness. The residue was crystallized from methanol to yield 9.7 g of (rac)-1-O-octadecyl-3-O-trityl-glycerol-2-tosylate. m.p. 54°C.

I.R. (Nujol): 1640, 1150 $cm^{-1}$

(2) To a solution of phenyl (5.75 g) in dry N,N-dimethylformamide (150 ml) was added potassium tert-butoxide (6.85 g) followed by stirring for 10 minutes at ambient temperature. To the resultant solution containing potassium phenoxide was added (rac)-1-O-octadecyl-3-O-trityl-glycerol-2-tosylate (15.03 g). The mixture was heated for 1.5 hours at 100°C, cooled in an ice bath, and poured into a mixture of benzene, ethyl acetate, and water. The organic layer was washed with 1% aqueous sodium hydroxide solution and water, dried, and evaporated. The residue was purified by column chromatography on silica gel (393 g, elution by a mixture of benzene and n-hexane, 1:4—1:2) to yield 10.1 g of (rac)-1-O-octadecyl-2-O-phenyl-3-O-trityl-glycerol as a colorless oil.

I.R. (Film): 3060, 2930, 2850, 1595, 1235, 1115 $cm^{-1}$

(3) To a solution of (rac)-1-O-octadecyl-2-O-phenyl-3-O-trityl-glycerol (10.0 g) in methylene chloride (100 ml) was added trifluoroacetic acid (11 ml) in one portion. After the yellow solution was stirred for 20 minutes at ambient temperature, ice water was added under vigorous stirring. The organic layer was washed with an aqueous sodium bicarbonate solution and water, dried, and evaporated under reduced pressure. The residue was treated with n-hexane to remove triphenylcarbinol by filtration. The filtrate was evaporated to dryness. The residue was purified by column chromatography on silica gel (120 g, elution by chloroform) to yield 4.48 g of (rac)-1-O-octadecyl-2-O-phenyl-glycerol as a waxy solid.

I.R. (Film): 3000, 2920, 1595, 1210 $cm^{-1}$

(4) To a solution of 2-bromoethyldichlorophosphate (3.30 g) in dry chloroform (6 ml) was added, dropwise over 20 minutes, a solution of (rac)-1-O-octadecyl-2-O-phenylglycerol (4.42 g) and triethylamine (1.92 g) in dry chloroform (6 ml) at 0 to 5°C. After the addition was completed, the mixture was allowed to warm to ambient temperature, stirred for 2 hours, and then cooled in an ice bath.

To the resultant thick solution containing (rac)-1-O-octadecyl-2-O-phenyl-glycerol-3-(2-bromoethyl)-chlorophosphate was added dropwise a mixture of water (5 ml) and pyridine (10 ml) below 15°C. The resulting solution was allowed to warm to ambient temperature followed by stirring for 30 minutes. The

solvents were then evaporated under reduced pressure. The residue was dissolved in aqueous sodium bicarbonate solution and diethyl ether. The aqueous phase was washed with diethyl ether, adjusted to pH 1 with 10% hydrochloric acid, and extracted twice with diethyl ether—ethyl acetate (50%).

The organic layer was washed with water, dried, and evaporated under reduced pressure to yield 6.7 g of (rac)-1-O-octadecyl-2-O-phenyl-glycerol-3-(2-bromoethyl)phosphate (oil).

I.R. (Film): 2920, 2850, 1595, 1235 $cm^{-1}$

(5)

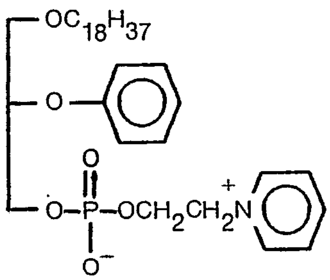

(rac)-1-O-Octadecyl-2-O-phenyl-glycerol-3-(2-(1-pyridinio)ethyl]phosphate (2.24 g) was obtained by reacting the above obtained compound of Example 4-(4) (3.05 g) with pyridine (9 ml) according to similar manners to those of Example 1 and 2. m.p. 196 to 200°C

I.R. (Nujol)®: 3380, 1635, 1595, 1585 $cm^{-1}$

N.M.R. ($CD_3OD$) ppm: 0.90 (3H, m), 1.02—1.72 (32H, m), 3.25—4.88 (11H, m), 6.77—7.38 (5H, m), 7.89—9.03 (5H, m)

| | | | |
|---|---|---|---|
| Anal. Calcd, for $C_{34}H_{56}NO_6P.2H_2O$: | C: 63.62, | H: 9.42, | N: 2.18 |
| Found: | C: 63.75, | H: 9.02, | N: 2.16 |

Example 5

(1) To a stirred suspension of (rac)-1-O-octadecyl-2-O-methyl-glycerol-3-(2-aminoethyl)phosphate (5.3 g) in dry chloroform (88 ml) was added bis(trimethylsilyl)acetamide (4.47 g) in one portion with stirring at ambient temperature followed by stirring for another one hour. The resulting solution was cooled in an ice bath and a solution of chloroacetyl chloride (1.37 g) in dry chloroform (22 ml) was dropwise added during a period of 5 minutes with stirring. After stirring for 3 hours at 5°C, the reaction mixture was washed with aqueous hydrochloric acid and with brine, dried, and evaporated under reduced pressure. The residue was crystallized from n-hexane to yield (rac)-1-O-octadecyl-2-O-methyl-glycerol-3-(2-chloroacetylamino-ethyl)phosphate. m.p. 67 to 70°C.

I.R. (Nujol®): 3300, 1640, 1220 $cm^{-1}$

(2)

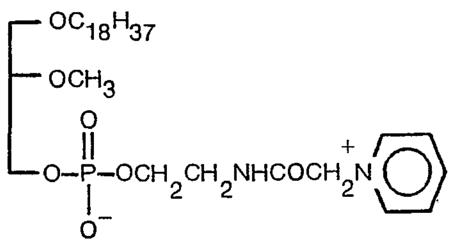

(rac)-1-O-Octadecyl-2-O-methyl-glycerol-3-[2-(1-pyridinioacetylamino)ethyl]phosphate (1.9 g) was obtained by reacting the above obtained compound (2.0 g) of Example 5-(1) with pyridine according to similar manners to those of Example 1 and 2. m.p. 224 to 226°C.

I.R. (Nujol®): 3350, 3200, 1675 $cm^{-1}$

N.M.R. ($CD_3OD$) ppm: 0.88 (3H, m), 1.12—1.49 (32H, m), 3.24—4.09 (11H, m), 3.46 (3H, s), 5.47 (2H, s), 8.14—8.96 (5H, m)

| | | | |
|---|---|---|---|
| Anal. Calcd. for $C_{31}H_{57}N_2O_7P.H_2O$: | C: 60.17, | H: 9.61, | N: 4.53 |
| Found: | C: 60.04, | H: 9.85, | N: 4.54 |

Example 6

(1) (rac)-1-O-Octadecyl-2-methylthio-3-O-trityl-1,3-propanediol (6.17 g) was obtained as an oil by reacting (rac)-1-O-octadecyl-3-O-trityl-glycerol-2-tosylate (7.3 g) with sodium methylmercaptide (10 ml, 15% in water) in a similar manner to that of Example 4-(2).

N.M.R. ($CCl_4$) ppm: 0.86 (3H, t, J=6Hz), 1.02—1.70 (32H, m), 1.96 (3H, s), 2.70 (1H, m), 3.25 (2H, d, J=6Hz), 3.30 (2H, t, J=6Hz), 3.55 (2H, d, J=6Hz), 7.00—7.56 (15H, m)

(2) To a solution of (rac)-1-O-octadecyl-2-methylthio-3-O-trityl-1,3-propanediol (2.8 g) in chloroform (22.5 ml) was dropwise added, during a period over 20 minutes, a solution of m-chloroperbenzoic acid (1.94 g) in chloroform (30 ml) under cooling at 5°C and the mixture was stirred for one hour at the same temperature. The precipitate was filtered off and washed with a small quantity of chloroform. The combined filtrates were washed with an aqueous sodium sulfite solution, an aqueous sodium bicarbonate solution, and water respectively. The organic layer was dried and evaporated to yield 3.19 g of (rac)-1-O-octadecyl-2-methylsulfonyl-3-O-trityl-1,3-propanediol as a thick oil.

I.R. (Film): 3030, 2920, 2850, 1595, 1350 $cm^{-1}$

(3) (rac)-1-O-Octadecyl-2-methylsulfonyl-1,3-propanediol (5.3 g) was obtained as an oil by reacting the above obtained compound of Example 6-(2) (8.92 g) with trifluoroacetic acid (10.9 ml) in a similar manner to that of Example 4-(3).

N.M.R. ($CCl_4$) ppm: 0.90 (3H, m), 1.07—1.78 (32H, m), 2.88 (3H, s), 2.97—3.33 (2H, m), 3.45 (2H, dd, J=6Hz), 3.82 (2H, d, J=6Hz), 3.72—4.10 (2H, m)

(4) (rac)-1-O-Octadecyl-2-methylsulfonyl-1,3-propanediol-3-(2-bromoethyl)phosphate (1.58 g) was prepared as an oil by reacting the above obtained compound of Example 6-(3) with 2-bromoethyldichlorophosphate (4.10 g) in a similar manner to that of Example 4-(4).

I.R. ($CHCl_3$): 3450, 3300, 2900, 1460, 1300, 1190 $cm^{-1}$

(5)

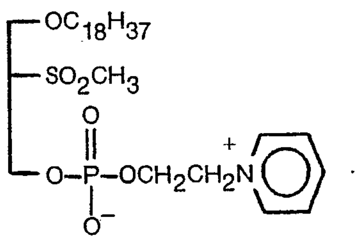

(rac)-1-O-Octadecyl-2-methylsulfonyl-1,3-propanediol-3-[2-(1-pyridinio)ethyl]phosphate (1.6 g) was obtained by reacting the above obtained compound of the Example 6-(4) (2.2 g) with pyridine (6.6 ml) according to similar manners to those of Examples 1 and 2. m.p. 168 to 172°C.

I.R. (Nujol®): 3350, 1465, 1295 $cm^{-1}$

N.M.R. ($CD_3OD$) ppm: 0.89 (3H, m), 1.08—1.43 (32H, m), 3.00 (3H, s), 3.18—4.89 (11H, m), 8.13—9.00 (5H, m)

| | | | |
|---|---|---|---|
| Anal. Calcd. for $C_{29}H_{54}NO_7PS.3/2H_2O$: | C; 56.29, | H, 2.26, | N: 9.28 |
| Found: | C: 56.23, | H: 2.21, | N: 9.59 |

Example 7

(1) (rac)-1-O-Octadecyl-2-phenylthio-3-O-trityl-1,3-propanediol (9.9 g) was obtained as an oil by reacting the above obtained compound Example 4-(1) (10.32 g) with thiophenol (3.08 g) in a similar manner to that of Example 4-(2).

I.R. (Film): 3050, 2920, 2850, 1445, 1100 $cm^{-1}$

(2) (rac)-1-O-Octadecyl-2-phenylthio-1,3-propanediol (6.7 g) was obtained as an oil by reacting the above obtained compound of Example 7-(1) (12.1 g) with trifluoroacetic acid (12.1 ml) in a similar manner to that of Example 4-(3).

I.R. (Film): 3450, 3020, 2930, 1463, 1213 $cm^{-1}$

(3) (rac)-1-O-Octadecyl-2-phenylthio-1,3-propanediol-3-(2-bromoethyl)phosphate (3.59 g) was obtained as an oil by reacting the above obtained compound of Example 7-(2) (5.55 g) with 2-bromoethyldichlorophosphate (4.0 g) in a similar manner to that of Example 4-(4).

I.R. ($CHCl_3$): 3400, 3010, 2920, 1210 $cm^{-1}$

(4)

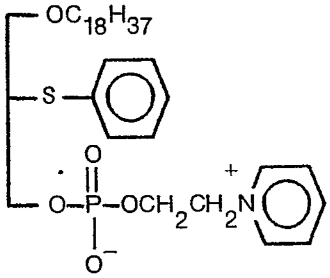

(rac)-1-O-Octadecyl-2-phenylthio-1,3-propanediol-3-[2-(1-pyridinio)ethyl]phosphate (2.5 g) was obtained by reacting the above obtained compound of Example 7-(3) (4.1 g) with pyridine (12.3 ml) according to similar manners to those of Examples 1 and 2. m.p. 194 to 196°C.

I.R. (Nujol®): 3350, 1630, 1580 $cm^{-1}$

N.M.R. ($CD_3OD$) ppm: 0.89 (3H, m), 1.04—1.63 (32H, m), 3.15—4.92 (11H, m), 7.05—7.51 (5H, m), 8.04—9.02 (5H, m)

| | | | |
|---|---|---|---|
| Anal. Calcd. for $C_{34}H_{56}NO_5PS.3/2H_2O$: | C: 62.94, | H: 9.16, | N: 2.16 |
| Found: | C: 63.16, | H: 9.19, | N: 2.17 |

## Example 8

(1) (rac)-1-O-Octadecyl-2-(1,3,4-thiadiazolyl-2-thio)-3-O-trityl-1,3-propanediol (4.61 g) was obtained as an oil by reacting (rac)-1-O-octadecyl-3-O-trityl-glycerol-2-tosylate (7.37 g) with 2-mercapto-1,3,4-thiadiazole (1.30 g) in a similar manner to that of Example 4-(2).

I.R. (Film): 2920, 2850, 1595, 1050 $cm^{-1}$

(2) (rac)-1-O-Octadecyl-2-(1,3,4-thiadiazolyl-2-thio)-1,3-propanediol (2.29 g) was obtained by reacting the above obtained compound of Example 8-(1) (4.10 g) with trifluoroacetic acid (4 ml) in a similar manner to that of Example 4-(3). m.p. 95°C.

I.R. (Nujol®): 3300, 1590 $cm^{-1}$

(3) (rac)-1-O-Octadecyl-2-(1,3,4-thiadiazolyl-2-thio)-1,3-propanediol-3-(2-bromoethyl)phosphate (2.16 g) was obtained as an oil by reacting the above obtained compound of Example 8-(2) (2.22 g) with 2-bromoethyldichlorophosphate (1.57 g) in a similar manner to that of Example 4-(4).

I.R. (Film): 2920, 2850, 1600, 1240 $cm^{-1}$

(4)

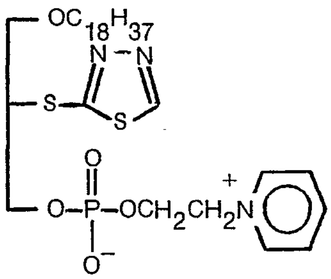

(rac)-1-O-Octadecyl-2-(1,3,4-thiadiazolyl-2-thio)-1,3-propanediol-3-[2-(1-pyridinio)ethyl]phosphate (1.08 g) was obtained by reacting the above obtained compound of the Example 8-(3) (2.10 g) with pyridine (20 ml) according to similar manners to those of Examples 1 and 2. m.p. 203 to 205°C.

I.R. (Nujol®): 3350, 1630, 1240 $cm^{-1}$

N.M.R. ($CD_3OD$) ppm: 0.90 (3H, t, J=5.5Hz), 1.04—1.64 (32H, m), 3.36—4.94 (11H, m), 8.14—9.10 (5H, m), 9.42 (1H, s)

| | | | |
|---|---|---|---|
| Anal. Calcd. for $C_{30}H_{52}N_3O_5PS_2.H_2O$: | C: 55.62, | H: 8.40, | N: 6.49 |
| Found: | C: 55.65, | H: 8.82, | N: 6.18 |

## Example 9

(1) (rac)-1-O-Octadecyl-2-chloro-3-O-trityl-1,3-propanediol (1.66 g) was obtained by reacting (rac)-1-O-octadecyl-3-O-trityl-glycerol-2-tosylate (3.0 g) with lithium chloride (0.85 g) in a similar manner to that of Example 4-(2). m.p. 68—71°C.

I.R. (Nujol®): 1595 $cm^{-1}$

(2) (rac)-1-O-Octadecyl-2-chloro-1,3-propanediol (3.3 g) was obtained by reacting the above obtained compound of Example 9-(1) (5.59 g) with trifluoroacetic acid (6 ml) in a similar manner to that of Example 4-(3). m.p. 40 to 41°C.

I.R. (Nujol®): 3320, 1465 $cm^{-1}$

(3) (rac)-1-O-Octadecyl-2-chloro-1,3-propanediol-3-(2-bromoethyl)phosphate (3.92 g) was obtained as a waxy solid by reacting the above obtained compound of Example 9-(2) (3.48 g) with 2-bromoethyldichlorophosphate (3.01 g) in a similar manner to that of Example 4-(4).

I.R. ($CHCl_3$): 2900, 2840, 2300, 1220 $cm^{-1}$

(4) 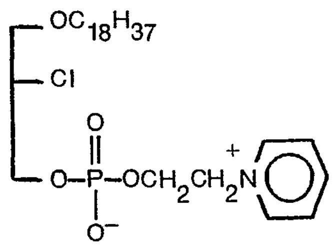

(rac)-1-O-Octadecyl-2-chloro-1,3-propanediol-3-[2-(1-pyridinio)ethyl]phosphate (2.73 g) was obtained by reacting the above obtained compound of Example 9-(3) (3.80 g) with pyridine (7 ml) according to similar manners to those of Examples 1 and 2. m.p. 203 to 205°C.

I.R. (Nujol®): 3350, 1240 $cm^{-1}$

N.M.R. ($CD_3OD$) ppm: 0.89 (3H, t, J=5Hz), 1.04—1.70 (32H, m), 3.34—4.44 (11H, m), 7.92—9.04 (5H, m)

| | | | |
|---|---|---|---|
| Anal. Calcd. for $C_{28}H_{51}NO_5ClP.H_2O$: | C: 59.40, | H: 9.44, | N: 2.47 |
| Found: | C: 59.17, | H: 9.59, | N: 2.51 |

Example 10

(1) (rac)-1-O-Octadecyl-2-azido-3-O-trityl-1,3-propanediol (12.2 g) was obtained by reacting (rac)-1-O-octadecyl-3-O-trityl-glycerol-2-tosylate (14.7 g) with sodium azide (3.9 g) in a similar manner to that of Example 4-(2). m.p. 47 to 49°C.

I.R. (Nujol®): 2100, 1595 $cm^{-1}$

(2) (rac)-1-O-Octadecyl-2-azido-1,3-propanediol (6.24 g) was obtained as a waxy solid by reacting the above obtained compound of Example 10-(1) (13.5 g) with trifluoroacetic acid (13.5 ml) in a similar manner to that of Example 4-(3).

I.R. ($CHCl_3$): 3450, 2900, 2850, 2200 $cm^{-1}$

(3) (rac)-1-O-Octadecyl-2-azido-1,3-propanediol-3-(2-bromoethyl)phosphate (4.90 g) was obtained as an amorphous solid by reacting the above obtained compound of Example 10-(2) (6.10 g) with 2-bromoethyl-dichlorophosphate (5.15 g) in a similar manner to that of Example 4-(4).

I.R. ($CHCl_3$): 2120, 1460, 1215 $cm^{-1}$

(4) A solution of (rac)-1-O-octadecyl-2-azido-1,3-propanediol-3-(2-bromoethyl)phosphate (6.7 g) in a mixture of ethyl acetate (40 ml), chloroform (30 ml), and acetic acid (20 ml) containing 10% palladium on charcoal (2.0 g) was hydrogenated at 294 KPa (3 atm.) hydrogen pressure for 8 hours. The catalyst was filtered off and washed with ethyl acetate. The combined filtrates were evaporated under reduced pressure. The residue was purified by column chromatography on silica gel (130 g, elution by $CHCl_3$, 5% methanol in chloroform, 10% methanol in chloroform, and chloroform:methanol:water, 65:25:4, by volume) followed by crystallization from methanol to yield 2.27 g of (rac)-1-O-octadecyl-2-amino-1,3-propanediol-3-(2-bromoethyl)phosphate. m.p. 210°C.

I.R. (Nujol®): 3350, 1220 $cm^{-1}$

(5) To a solution of the above obtained compound of Example 10-(4) (1.65 g) and dry pyridine (1.5 g) in dry methylenechloride (20 ml) was added, dropwise over 20 minutes; a solution of methyl chloroformate (0.34 g) in dry methylene chloride (5 ml) at 5°C. After 3 hours, the solution was allowed to stand for 17 hours at ambient temperature, and then washed with aqueous hydrochloric acid and water, dried, and evaporated. The residue was purified by column chromatography on silica gel (30 g, elution by chloroform) to yield 1.20 g of (rac)-1-O-octadecyl-2-ethoxycarbonylamino-1,3-propanediol-3-(2-bromoethyl)phosphate as an amorphous solid.

I.R. ($CHCl_3$): 3270, 1715, 1250 $cm^{-1}$

(6) 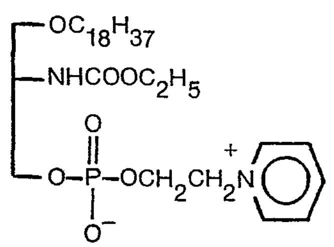

(rac)-1-O-Octadecyl-2-ethoxycarbonylamino-1,3-propanediol-3-[2-(1-pyridinio)ethyl]phosphate (0.60 g) was obtained by reacting the above obtained compound of Example 10-(5) (1.15 g) with pyridine (6 ml) according to similar manners to those of Examples 1 and 2. m.p. 175 to 176°C (dec.).

I.R. (Nujol®): 3470, 1710, 1635, 1240 $cm^{-1}$
N.M.R. ($CD_3OD$) ppm: 0.89 (3H, t, J=5.5Hz), 1.10—1.70 (35H, m), 3.32—4.88 (13H, m), 8.08—8.98 (5H, m)

| | | | |
|---|---|---|---|
| Anal. Calcd. for $C_{31}H_{57}N_2O_7P.3/2H_2O$: | C: 59.31, | H: 9.63; | N: 4.46 |
| Found: | C: 59.57, | H: 9.64, | N: 3.97 |

Example 11

(1) To a suspension of (rac)-1-O-octadecyl-2-amino-1,3-propanediol-3-(2-bromoethyl)phosphate (2.20 g) in dry methylene chloride (30 ml) was added bis(trimethylsilyl)acetamide (2.0 g) in one portion. After the mixture was stirred for 5 hours at ambient temperature, a solution of methyl isocyanate (0.79 g) in dry methylene chloride (2 ml) was added therein. After standing overnight, the mixture was washed with aqueous hydrochloric acid and twice with water, dried, and evaporated. The residue was triturated in methanol to yield 1.66 g of (rac)-1-O-octadecyl-2-(N′-methylureido)-1,3-propanediol-3-(2-bromoethyl)-phosphate. m.p. 95°C.
I.R. (Nujol®): 3320, 1630, 1230 $cm^{-1}$

(2)

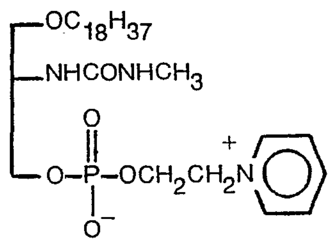

(rac)-1-O-Octadecyl-2-(N′-methylureido)-1,3-propanediol-3-[2-(1-pyridinio)ethyl]phosphate (0.65 g) was obtained by reacting the above obtained compound of Example 11-(1) (1.64 g) with pyridine (5 ml) according to similar manners to those of Examples 1 and 2. m.p. 189°C (dec.)
I.R. (Nujol®): 3330, 1635, 1235 $cm^{-1}$
N.M.R. ($CD_3OD$) ppm: 0.90 (3H, t, J=5.5Hz), 1.04—1.70 (32H, m), 2.67 (3H, s), 3.32—4.88 (11H, m), 8.10—9.00 (5H, m)

| | | | |
|---|---|---|---|
| Anal. Calcd. for $C_{30}H_{56}N_3O_6P.H_2O$: | C: 59.67, | H: 9.68, | N: 6.96 |
| Found: | C: 59.43, | H: 9.99, | N: 6.79 |

Example 12

(1) (rac)-1-O-(Octadec-cis-9-enyl)-2-O-methyl-3-O-trityl-glycerol (9.4 g) was obtained as an oil by methylation of (rac)-1-O-(octadec-cis-9-enyl)-3-O-trityl-glycerol (8.94 g) with methyl iodide (2.83 g).
I.R. (Film): 3050, 2920, 2850, 1445 $cm^{-1}$

(2) (rac)-1-O-(Octadec-cis-9-enyl)-2-O-methyl-glycerol (4.08 g) was obtained as an oil by reacting the above obtained compound of Example 12-(1) (9.4 g) with trifluoroacetic acid (9.4 ml) in a similar manner to that of Example 4-(3).
I.R. [Film]: 3400, 2900, 2850, 1460 $cm^{-1}$

(3) (rac)-1-O-(Octadec-cis-9-enyl)-2-O-methyl-glycerol-3-(2-bromoethyl)phosphate (5.39 g) was obtained by reacting the above obtained compound of Example 12-(2) (4.0 g) with 2-bromoethyldichloro-phosphate (4.07 g) in a similar manner to that of Example 4-(4).
I.R (Film): 2900, 2850, 1460, 1230 $cm^{-1}$

(4)

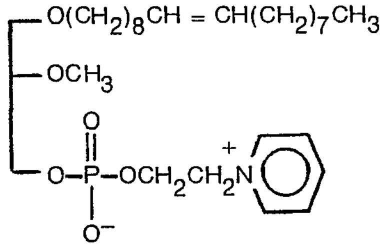

(rac)-1-O-(Octadec-cis-9-enyl)-2-O-methyl-glycerol-3-[2-(1-pyridino)ethyl]phosphate (1.9 g) was obtained as an oil by reacting the above obtained compound of Example 12-(3) (2.2 g) with pyridine according to similar manners to those of Example 1 and 2.
I.R. (Film): 3350, 3030, 2900, 2820, 1630, 1460, 1230 $cm^{-1}$
N.M.R. ($CD_3OD$) ppm: 0.89 (3H, m), 1.08—1.71 (24H, m), 1.81—2.23 (4H, m), 3.41 (3H, s), 3.45—5.39 (13H, m), 8.08—8.97 (5H, m)

| | | | |
|---|---|---|---|
| Anal. Calcd. for $C_{29}H_{52}NO_6P.3/2H_2O$: | C: 61.24, | H: 9.75, | N: 2.46 |
| Found: | C: 61.31, | H: 9.81, | N: 2.40 |

Example 13

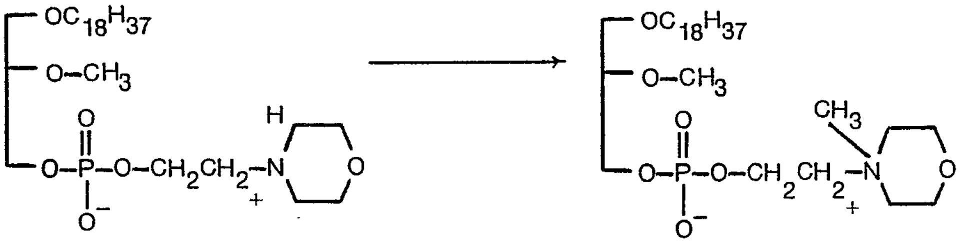

A mixture of (rac)-1-O-octadecyl-2-O-methyl-glycerol-3-(2-morpholinoethyl)phosphate (3.05 g), cyclohexylamine (1.17 g) and methyl iodide (6.64 g) in ethanol (45 ml) was refluxed for 7 hours. The reaction mixture was evaporated under reduced pressure and the residue was triturated in acetone. The obtained powder was dissolved in 90% aqueous methanol (50 ml), and the solution was treated with silver acetate (4.02 g) for 30 minutes at room temperature. The precipitate was filtered off and washed with methanol. The filtrate and washings were combined and evaporated to dryness. The residue was purified by column chromatography on silica gel (60 g, elution by chloroform-methanol-water, 65:25:4) to give 1.62 g of (rac)-1-O-octadecyl-2-O-methyl-glycerol-3-[2-(N-methyl-4-morpholinio)ethyl]phosphate, which was recrystallized from chloroform-acetone. yield 1.19 g (hygroscopic solid). m.p. 58°C.

I.R. (Nujol®): 3350, 1625, 1220 $cm^{-1}$

| | | | |
|---|---|---|---|
| Anal. Calcd. for $C_{29}H_{60}O_7NP.3/2H_2O$: | C: 58.75, | H: 10.71, | N: 2.36 |
| Found: | C: 58.38, | H: 10.79, | N: 2.37 |

Example 14

The following compounds were obtained according to a similar manner to that of Example 13.

(1)

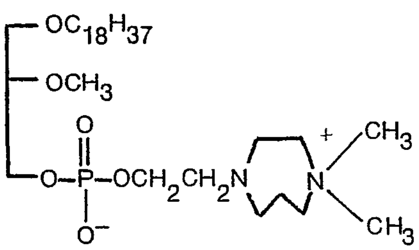

(rac)-1-O-Octadecyl-2-O-methyl-glycerol-3-[2-(4-dimethyl-1-homopiperazinio)ethyl]phosphate. m.p. 210 to 214°C.

I.R. (Nujol®): 3330, 1240, 1095 $cm^{-1}$

N.M.R. ($CD_3OD$) ppm: 0.90 (3H, m), 1.09—2.22 (34H, m), 2.73—4.79 (19H, m), 3.17 (6H, s), 3.46 (3H, s)

| | | | |
|---|---|---|---|
| Anal. Calcd. for $C_{31}H_{65}N_2O_6P.5/4H_2O$: | C: 60.51, | H: 11.01, | H: 4.55 |
| Found: | C: 60.53, | H: 11.07, | N: 4.43 |

(2) (rac)-1-O-Octadecyl-2-O-ethyl-glycerol-3-[2-(4-methyl-1-piperazinyl)ethyl]phosphate. m.p. 115°C.

I.R. (Nujol®): 3400, 1645, 1215, 1090, 1055 $cm^{-1}$

(3) (rac)-1-O-Hexadecyl-2-O-methyl-glycerol-3-[2-(4-methyl-1-piperazinyl)ethyl]phosphate.

I.R. (Nujol®): 3350, 1220 $cm^{-1}$

(4) (rac)-1-O-Octadecyl-2-O-methyl-glycerol-3-[2-(4-ethyl-1-piperazinyl)ethyl]phosphate.

I.R. (Nujol®): 3350, 1210, 1050 $cm^{-1}$

(5) (rac)-1-O-Octadecyl-2-O-methyl-glycerol-3-[2-[4-(2-hydroxyethyl)-1-piperazinyl]ethyl]phosphate. m.p. 196 to 199°C.

I.R. (Nujol®): 3120, 2200, 1210, 1050 $cm^{-1}$

(6) (rac)-1-O-Hexadecyl-2-O-benzyl-glycerol-3-[2-(4-methyl-1-piperazinyl)ethyl]phosphate.

I.R. (Film): 3350, 2700, 1640, 1200, 1050 $cm^{-1}$

(7) (rac)-1-O-(Tetradecyl)-2-O-methyl-glycerol-3-[2-(4-methyl-1-piperazinyl)ethyl]phosphate.

I.R. (Film): 3400, 2800, 1650, 1220, 1050 $cm^{-1}$

(8) (rac)-1-O-Tridecyl-2-O-methyl-glycerol-3-[2-(4-methyl-1-piperazinyl)ethyl]phosphate.

Example 15

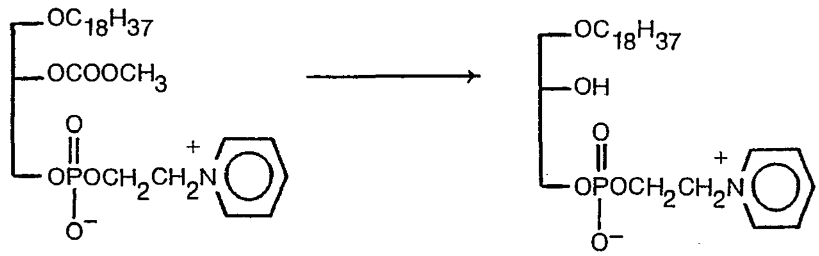

To a solution of (rac)-1-O-octadecyl-2-O-methoxycarbonyl-glycerol-3-(2-pyridinioethyl)phosphate (1.0 g) in methyl alcohol (20 ml) was dropwise added during a period over one minute 1N-aqueous sodium hydroxide solution (2 ml) at ambient temperature. After stirring for 1.5 hours at the same temperature, the mixture was evaporated under reduced pressure. The resultant residue was dissolved in a mixture of chloroform-methyl alcohol-water (20 ml, 1:2:1 by volume) and passed through a mixed ion-exchange column (Amberlite® IRC-50($H^+$) 3.7 g, IR-45($OH^-$) 7.3 g). After washing the column with the same solvents, the combined eluates were evaporated to dryness. Purification of the obtained residue was carried out by column chromatography on silica gel (54 g, elution by chloroform-methyl alcohol-water, 65:25:4, by volume). The combined eluates containing the desired product were evaporated to dryness and the residue was crystallized from a mixture of chloroform and acetone to yield 0.53 g of (rac)-1-O-octadecyl-glycerol-3-[2-(1-pyridinio)ethyl]phosphate as a colorless solid. m.p. 150°C (dec.).

I.R. (Nujol): 3410, 1635, 1235 $cm^{-1}$

N.M.R. ($CD_3OD$) ppm: 0.9 (3H, t, J=5Hz), 1.0—1.8 (32H, m), 3.08—4.88 (11H, m), 8.12—8.94 (5H, m)

| | | | |
|---|---|---|---|
| Anal. Calcd. for $C_{28}H_{52}NO_6P.2H_2O$: | C: 59.44, | H: 9.97, | N: 2.48 |
| Found: | C: 59.11, | H: 9.66, | N: 2.43 |

Example 16

(1) (rac)-1-O-Octadecyl-2-methylthio-1,3-propanediol (2.68 g) was obtained as a waxy solid by reacting (rac)-1-O-octadecyl-2-methylthio-3-O-trityl-1,3-propanediol (5.59 g) with trifluoroacetic acid (5.6 ml) in a similar manner to that of Example 4-(3).

I.R. (Film): 3300, 1460 $cm^{-1}$

(2)

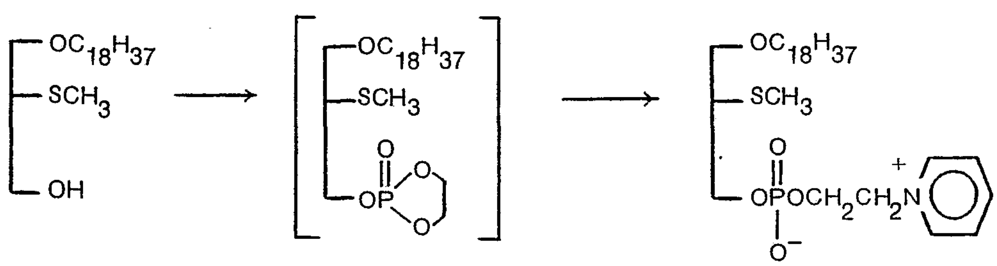

To a solution of (rac)-1-O-octadecyl-2-methylthio-1,3-propanediol (2.68 g) and triethylamine (0.87 g) in dry benzene (35 ml) was added a solution of 2-chloro-2-oxo-1,3,2-dioxaphospholidine (1.22 g) in dry benzene (5 ml) during 5 minutes at 5°C under stirring. After the addition, the mixture was allowed to warm to ambient temperature and stirred for 2 hours further. The precipitate was filtered off and washed with a small quantity of dry benzene. The combined filtrate and washings were evaporated under reduced pressure and the residue was dissolved in dry pyridine (6 ml).

The resultant mixture was heated for 1.5 hours at 100°C and then evaporated to dryness. The residue was triturated with acetone to give a crude product, which was purified by column chromatography on silica gel (80 g. elution by a mixture of chloroform-methyl alcohol-water, 65:25:4, by volume) followed by recrystallization from a mixture of chloroform and acetone to yield 0.55 g of (rac)-1-O-octadecyl-2-methyl-thio-1,3-propanediol-3-[2-(1-pyridinio)ethyl]phosphate. m.p. 194—195°C.

I.R. (Nujol®): 3350, 1635, 1240 $cm^{-1}$

N.M.R. ($CD_3OD$) ppm: 0.91 (3H, t, J=5.5Hz), 1.04—1.64 (32H, m), 2.14 (3H, s), 2.89 (1H, m), 3.38—4.90 (10H, m), 8.06—9.06 (5H, m)

| | | | |
|---|---|---|---|
| Anal. Calcd. for $C_{29}H_{54}NO_5PS.5/2H_2O$: | C: 59.35, | H: 9.79, | N: 2.38 |
| Found: | C: 59.03, | H: 9.59, | N: 2.28 |

Example 17

The following compounds were obtained according to a similar manner to that of Example 16.

(1) (rac)-1-O-Octadecyl-2-O-ethyl-glycerol-3-[2-(4-methyl-1-piperazinyl)ethyl]phosphate.
I.R. (Nujol®): 3400, 1645, 1215, 1090, 1055 $cm^{-1}$

(2) (rac)-1-O-Hexadecyl-2-O-methyl-glycerol-3-[2-(4-methyl-1-piperazinyl)ethyl]phosphate.
I.R. (Nujol®): 3350, 1220 $cm^{-1}$

(3) (rac)-1-O-Octadecyl-2-O-methyl-glycerol-3-[2-(4-ethyl-1-piperazinyl)ethyl]phosphate.
I.R. (Nujol®): 3350, 1210, 1050 $cm^{-1}$

(4) (rac)-1-O-Octadecyl-2-O-methyl-glycerol-3-[2-(1-piperazinyl)ethyl]phosphate.
I.R. (Nujol®): 3350, 1625, 1220, 1070 $cm^{-1}$

(5) (rac)-1-O-Octadecyl-2-O-methyl-glycerol-3-[2-(4-hydroxypiperidino)ethyl]phosphate.
I.R. (Nujol®): 3200, 1215, 1055 $cm^{-1}$

(6) (rac)-1-O-Octadecyl-2-O-methyl-glycerol-3-[2-(4-methylpiperidino)ethyl]phosphate.
I.R. (Nujol®): 3400, 1220, 1040 $cm^{-1}$

(7) (rac)-1-O-Octadecyl-2-O-methyl-glycerol-3-[2-[4-(2-hydroxyethyl)-1-piperazinyl]ethyl]phosphate.
I.R. (Nujol®): 3120, 2200, 1210, 1050 $cm^{-1}$

(8) (rac)-1-O-Hexadecyl-2-O-benzyl-glycerol-3-[2-(4-methyl-1-piperazinyl)ethyl]phosphate.
I.R. (Film): 3350, 2700, 1640, 1200, 1050 $cm^{-1}$

(9) (rac)-1-O-Octadecyl-2-O-methyl-glycerol-3-[2-(4-methyl-1-pyridinio)ethyl]phosphate.
I.R. (Nujol®): 3350, 1640, 1240 $cm^{-1}$

(10) (rac)-1-O-Octadecyl-2-O-methyl-glycerol-3-[2-[3-methyl-1-pyridinio]ethyl]phosphate.
I.R. ($CHCl_3$): 3650, 3300, 2850, 1635 $cm^{-1}$

(11) (rac)-1-O-Octadecyl-2-O-methyl-glycerol-3-[2-(4-dimethylamino-1-pyridinio)ethyl]phosphate.
I.R. (Nujol®): 3350, 1650, 1570, 1230 $cm^{-1}$

(12) (rac)-1-O-Octadecyl-2-O-methyl-glycerol-3-[2-(1-pyridazinio)ethyl]phosphate.
I.R. (Nujol®): 3350, 1600, 1245 $cm^{-1}$

(13) (rac)-1-O-Octadecyl-2-O-methyl-glycerol-3-[2-(1-imidazolyl)ethyl]phosphate.
I.R. (Nujol®): 3100, 1260, 1120, 1085, 1050, 930 $cm^{-1}$

(14) (rac)-1-O-Octadecyl-2-O-methyl-glycerol-3-(2-piperidinoethyl)phosphate.
I.R. (Nujol®): 3400, 1660, 1230, 1120, 1085, 1070, 1055, 1020 $cm^{-1}$

(15) (rac)-1-O-Octadecyl-2-O-methyl-glycerol-3-[2-(N-methyl-4-morpholinio)ethyl]phosphate, hygroscopic solid.
I.R. (Nujol®): 3350, 1625, 1220 $cm^{-1}$

(16) (rac)-1-O-Octadecyl-2-O-methyl-glycerol-3-[2-(4-methyl-1-piperazinyl)ethyl]phosphate.
I.R. (Nujol®): 3350, 1650, 1210, 1050 $cm^{-1}$

(17) (rac)-1-O-Octadecyl-2-O-methyl-glycerol-3-[2-(3-chloro-1-pyridinio)ethyl]phosphate.
I.R. (Nujol®): 3350, 1625, 1240 $cm^{-1}$

(18) (rac)-1-O-Octadecyl-2-O-methyl-glycerol-3-[2-(1-methyl-2-pyrazolio)ethyl]phosphate.
I.R. (Nujol®): 3370, 1675 (s), 1240 $cm^{-1}$

(19) (rac)-1-O-Octadecyl-2-O-ethyl-glycerol-3-[2-(1-pyridinio)ethyl]phosphate.
I.R. ($CHCl_3$): 3300, 1635, 1240 $cm^{-1}$

(20) (rac)-1-O-Octadecyl-2-O-benzyl-glycerol-3-[2-(1-pyridinio)ethyl]phosphate.
I.R. (Nujol®): 3370, 1230 $cm^{-1}$

(21) (rac)-1-O-Octadecyl-2-O-methyl-glycerol-3-[2-(1-methyl-1,2,4-triazol-4-io)ethyl]phosphate.
I.R. ($CHCl_3$): 3280, 3130, 2900, 2850, 1580 $cm^{-1}$

(22) (rac)-1-O-Octadecyl-2-O-methyl-glycerol-3-[2-(3-thiazolio)ethyl]phosphate.
I.R. ($CHCl_3$): 3300, 2900, 2850, 1660, 1460 $cm^{-1}$

(23) (rac)-1-O-Octadecyl-2-O-methyl-glycerol-3-[2-(3-ethoxycarbonyl-1-pyridinio)ethyl]phosphate.
I.R. ($CHCl_3$): 3300, 2900, 2850, 1740, 1460 $cm^{-1}$

(24) (rac)-1-O-Octadecyl-2-O-methyl-glycerol-3-[2-(3-hydroxymethyl-1-pyridinio)ethyl]phosphate.
I.R. ($CHCl_3$): 3200, 2900, 2850, 1635, 1460 $cm^{-1}$

(25) (rac)-1-O-Octadecyl-2-O-methyl-glycerol-3-[2-(1-methyl-3-imidazolio)ethyl]phosphate.
I.R. ($CHCl_3$): 3300, 2900, 2850, 1570, 1460 $cm^{-1}$

(26) (rac)-1-O-Hexadecyl-2-O-benzyl-glycerol-3-[2-(1-pyridinio)ethyl]phosphate.
I.R. (Nujol®): 3350, 3050, 1640, 1230, 1100, 1070 $cm^{-1}$

(27) (rac)-1-O-Hexadecyl-2-O-methyl-glycerol-3-[2-(1-pyridinio)ethyl]phosphate.
I.R. (Nujol®): 3370, 1640, 1245, 1130, 1080 $cm^{-1}$

(28) (rac)-1-O-Octadecyl-2-O-methyl-glycerol-3-[2-(1-homopiperazinyl)ethyl]phosphate. waxy solid.
I.R. (Nujol®): 3400, 1620, 1210, 1050 $cm^{-1}$

(29) (rac)-1-O-Octadecyl-1,3-propanediol-3-[2-(1-pyridinio)ethyl]phosphate.
I.R. (Nujol®): 3390, 1685, 1640, 1490 $cm^{-1}$

(30) (rac)-1-O-Octadecyl-2-O-methyl-glycerol-3-[2-(4-dimethyl-1-homopiperazinio)ethyl]phosphate.
I.R. (Nujol®): 3330, 1240, 1095 $cm^{-1}$

(31) (rac)-1-O-Tetradecyl-2-O-methyl-glycerol-3-[2-(4-methyl-1-piperazinyl)ethyl]phosphate.
I.R. (Film): 3400, 2800, 1650, 1220, 1050 $cm^{-1}$

(32) (rac)-1-O-Tridecyl-2-O-methyl-glycerol-3-[2-(4-methyl-1-piperazinyl)ethyl]phosphate.

(33) (rac)-1-O-Octadecyl-2-O-methyl-glycerol-3-[2-(1-pyridinio)ethyl]phosphate.
I.R. (Nujol®): 3380, 1590, 1220, 1080 $cm^{-1}$

(34) (rac)-1-O-Octadecyl-2-O-methyl-glycerol-3-(2-morpholinoethyl)phosphate.
I.R. (Nujol®): 3350, 1220, 1115 $cm^{-1}$

(35) (rac)-1-O-Octadecyl-2-O-phenyl-glycerol-3-[2-(1-pyridinio)ethyl]phosphate.
I.R. (Nujol®): 3380, 1635, 1595, 1585 $cm^{-1}$

(36) (rac)-1-O-Octadecyl-2-methylsulfonyl-1,3-propanediol-3-[2-(1-pyridinio)ethyl]phosphate.
I.R (Nujol®): 3350, 1465, 1295 $cm^{-1}$

(37) (rac)-1-O-Octadecyl-2-phenylthio-1,3-propanediol-3-[2-(pyridinio)ethyl]phosphate.
I.R. (Nujol®): 3350, 1630, 1580 $cm^{-1}$

(38) (rac)-1-O-Octadecyl-2-(1,3,4-thiadiazolyl-2-thio)-1,3-propanediol-3-[2-(1-pyridinio)ethyl]phosphate.
I.R. (Nujol®): 3350, 1630, 1240 $cm^{-1}$

(39) (rac)-1-O-Octadecyl-2-chloro-1,3-propanediol-3-[2-(1-pyridinio)ethyl]phosphate.
I.R. (Nujol®): 3350, 1240 $cm^{-1}$

(40) (rac)-1-O-Octadecyl-2-ethoxycarbonylamino-1,3-propanediol-3-[2-(1-pyridinio)ethyl]phosphate.
I.R. (Nujol®): 3470, 1710, 1635, 1240 $cm^{-1}$

(41) (rac)-1-O-Octadecyl-2-(N'-methylureido)-1,3-propanediol-3-[2-(1-pyridino)ethyl]phosphate.
I.R. (Nujol®): 3330, 1635, 1235 $cm^{-1}$

(42) (rac)-1-O-(Octadec-cis-9-enyl)-2-O-methyl-glycerol-3-[2-(1-pyridinio)ethyl]phosphate.
I.R. (Film): 3350, 3030, 2900, 2820, 1630, 1460, 1230 $cm^{-1}$

**Claims**

1. A compound of the formula

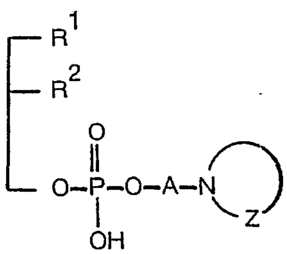

(I)

wherein

$R^1$ is $C_1$—$C_{25}$-alkoxy or -alkenyloxy;

$R^2$ is hydrogen, halogen, hydroxy, lower alkoxy, ar(lower)alkoxy, aryloxy, lower alkylthio, arylthio, thiadiazolylthio, lower alkylsulfonyl, lower alkoxycarbonylamino or lower alkylureido;

A is lower alkylene optionally interrupted by a —NHCO— group; and

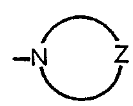

is unsaturated or saturated 5—7 membered heterocyclic group which may contain additional N, O or S atom(s), and may have 1 to 3 substituent(s) selected from lower alkyl, lower alkylamino, lower alkoxy, halogen, hydroxy, carboxy, lower alkoxycarbonyl and hydroxy(lower)alkyl groups;

provided that $R^1$ is alkoxy having 15 or more carbon atoms when

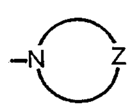

is a pyridinio group,
and pharmaceutically acceptable salts thereof.

2. A compound of claim 1, wherein

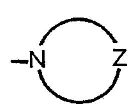

is saturated 5—7 membered heterocyclic group which may contain additional N, O or S atom(s), and may have 1 to 3 substituent(s) selected from lower alkyl, lower alkylamino, lower alkoxy, halogen, hydroxy, carboxy, lower alkoxycarbonyl and hydroxy(lower)alkyl groups.

3. A compound of claim 2, wherein $R^1$ is alkoxy; $R^2$ is lower alkoxy or ar(lower)alkoxy; A is lower alkylene; and

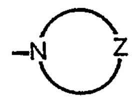

is saturated 6 or 7 membered heterocyclic group which may contain additional N or O atom(s) and may have 1 to 3 substituent(s) selected from lower alkyl, hydroxy and hydroxy(lower)alkyl groups.

4. A compound of claim 3, wherein $R^2$ is lower alkoxy.

5. A compound of claim 4, wherein

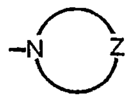

is morpholino, piperazinyl, piperidino or homopiperazinyl which may have 1 to 2 substituent(s) selected from lower alkyl, hydroxy and hydroxy(lower)alkyl groups.

6. (rac)-1-O-Octadecyl-2-O-methyl-glycerol-3-[2-(N-methylmorpholino)ethyl]phosphate.

7. (rac)-1-O-Hexadecyl-2-O-methyl-glycerol-3-[2-(4-methyl-1-piperazinyl)ethyl]phosphate.

8. (rac)-1-O-Octadecyl-2-O-methyl-glycerol-3-[2-(4-methyl-1-piperazinyl)ethyl]phosphate.

9. A compound of claim 3, wherein $R^2$ is ar(lower)alkoxy.

10. (rac)-1-O-Hexadecyl-2-O-benzyl-glycerol-3-[2-(4-methyl-1-piperazinyl)ethyl]phosphate.

11. A compound of claim 1, wherein

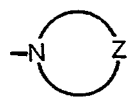

is unsaturated 5—7 membered heterocyclic group which may contain additional N, O or S atom(s), and may have 1 to 3 substituent(s) selected from lower alkyl, lower alkylamino, lower alkoxy, halogen, hydroxy, carboxy, lower alkoxycarbonyl and hydroxy(lower)alkyl groups.

12. A compound of claim 11, wherein $R^1$ is alkoxy having 15 or more carbon atoms or alkenyloxy; $R^2$ is hydrogen, halogen, hydroxy, lower alkoxy, ar(lower)alkoxy, aryloxy, lower alkylthio, arylthio, thiadiazolylthio, lower alkylsulfonyl, lower alkoxycarbonylamino or lower alkylureido; A is lower alkylene optionally interrupted by a —NHCO— group; and

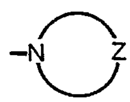

is unsaturated 5 or 6 membered heterocyclic group which may contain additional N or S atom(s) and may have 1 or 2 substituent(s) selected from lower alkyl, lower alkylamino, halogen, lower alkoxycarbonyl and hydroxy(lower)alkyl groups.

13. A compound of claim 12, wherein $R^1$ is alkoxy having 15 or more carbon atoms.

14. A compound of claim 13, wherein $R^2$ is lower alkoxy.

15. (rac)-1-O-Octadecyl-2-O-methyl-glycerol-3-[2-(1-pyridinio)ethyl]phosphate.

16. (rac)-1-O-Octadecyl-2-O-methyl-glycerol-3-[2-(3-methyl-1-pyridinio)ethyl]phosphate.

17. (rac)-1-O-Octadecyl-2-O-methyl-glycerol-3-[2-(1-pyridinioacetylamino)ethyl]phosphate.

18. (rac)-1-O-Octadecyl-2-O-methyl-glycerol-3-[2-(4-methyl-1-pyridinio)ethyl]phosphate.

19. A compound of claim 13, wherein $R^2$ is ar(lower)alkoxy.

20. (rac)-1-O-Hexadecyl-2-O-benzyl-glycerol-3-[2-(1-pyridinio)ethyl]phosphate.

21. A compound of claim 13, wherein $R^2$ is hydroxy.

22. (rac)-1-O-Octadecyl-glycerol-3-[2-(1-pyridinio)ethyl]phosphate.

23. A compound of claim 13, wherein $R^2$ is thiadiazolylthio.

24. (rac)-1-O-Octadecyl-2-(1,3,4-thiadiazol-2-ylthio)1,3-propanediol-3-[2-(1-pyridinio)ethyl]phosphate.

25. A compound of claim 13, wherein $R^2$ is lower alkoxycarbonylamino.

26. (rac)-1-O-Octadecyl-2-ethoxycarbonylamino-1,3-propanediol-3-[2-(1-pyridinio)ethyl]phosphate.

27. A process for preparing a compound of formula (I) as claimed in claim 1 and pharmaceutically acceptable salts thereof, which comprises

(1) reacting a compound of the formula

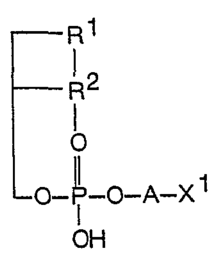

(II)

wherein $R^1$, $R^2$ and A are each as defined in claim 1, and $X^1$ is an acid residue, or its salt with a compound of the formula:

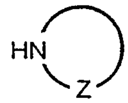 (III)

wherein

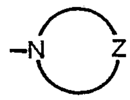

is as defined in claim 1, or its salt to provide a compound of the formula:

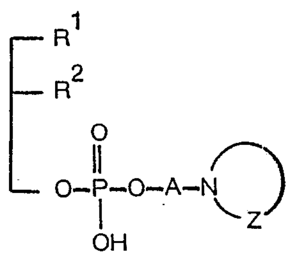 (I)

wherein $R^1$, $R^2$, A and

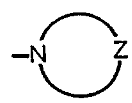

are each as defined above or its salt, or

(2) reacting a compound of the formula:

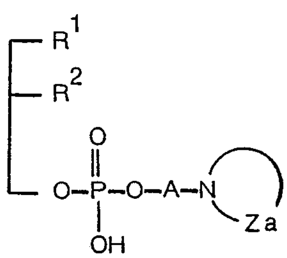 (Ib)

wherein $R^1$, $R^2$ and A are each as defined above, and

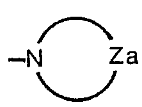

is unsaturated or saturated 5—7 membered heterocyclic group which may contain additional N, O or S atom(s), and may have 1 to 2 substituent(s) selected from lower alkyl, lower alkylamino, lower alkoxy, halogen, hydroxy, carboxy, lower alkoxycarbonyl and hydroxy(lower)alkyl groups, or its salt with a compound of the formula:

$$X^2—R^3 \quad (IV)$$

wherein $R^3$ is lower alkyl and $X^2$ is an acid residue, to provide a compound of the formula:

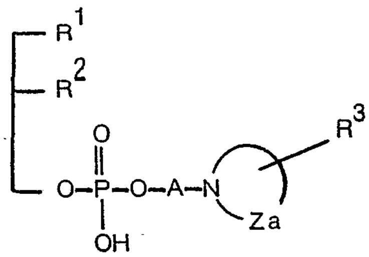

(Ia)

wherein $R^1$, $R^2$, A, $R^3$ and

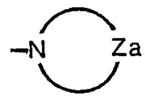

are each as defined above, or its salt, or

(3) subjecting a compound of the formula:

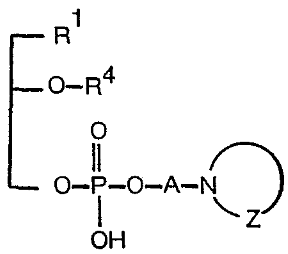

(V)

wherein $R^1$, A and

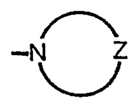

are each as defined above, and $R^4$ is a protective group for hydroxy, or its salt to an elimination reaction of the protective group to provide a compound of the formula:

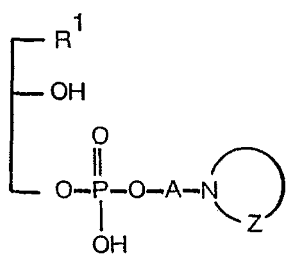

(Ic)

wherein $R^1$, A and

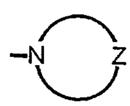

are each as defined above, or its salt, or

(4) reacting a compound of the formula:

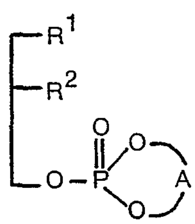

(VI)

wherein $R^1$, $R^2$ and A are each as defined above, or its salt with a compound of the formula:

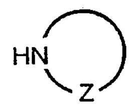

(III)

wherein

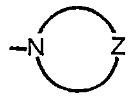

is as defined above, or its salt to provide a compound of the formula:

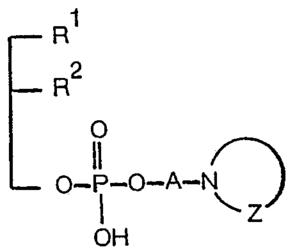

(I)

wherein $R^1$, $R^2$, A and

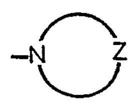

are each as defined above, or its salt.

28. A pharmaceutical composition comprising an effective amount of a compound of formula (I) as claimed in claim 1 or pharmaceutically acceptable salts thereof, with a pharmaceutically acceptable, substantially nontoxic carrier or excipient.

29. A compound of formula (I) as claimed in claim 1 for use as an active therapeutic substance.

30. A compound of formula (I) as claimed in claim 1 for use as an active substance combating cancer.

**Patentansprüche:**

1. Verbindung der Formel

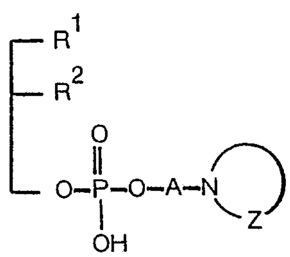

(I)

worin

$R^1$ $C_1$—$C_{25}$-lkoxy- oder -alkenyloxy ist;

$R^2$ Wasserstoff, Halogen, Hydroxy, niedrig-Alkoxy, Ar-(niedrig)-alkoxy, Aryloxy, niedrig-Alkylthio, Arylthio, Thiadiazolylthio, niedrig-Alkylsulfonyl, niedrig-Alkoxycarbonalamino oder niedrig-Alkylureido ist;

A niedrig-Alkylen, gegebenenfalls unterbrochen durch eine —NHCO—Gruppe ist; und

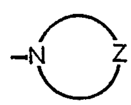

eine ungesättigte oder gesättigte 5-7-gliedrige heterocyclische Gruppe ist, die susätzliche N-, O- oder S-Atom(e) enthalten kann und 1 bis 3 Substituenten haben kann, ausgewählt aus niedrig-Alkyl-, niedrig-Alkylamino-, niedrig-Alkoxy-, Halogen-, Hydroxy-, Carboxy-, niedrig-Alkoxycarbonyl- und Hydroxy-niedrig-alkylgruppen;

vorausgesetzt daß $R^1$ Alkoxy mit 15 oder mehr Kohlenstoffatomen ist, wenn

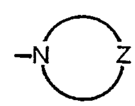

eine Pyrdiniogruppe ist,
und pharmazeutisch brauchbare Salze davon.

2. Verbindung nach Anspruch 1, worin

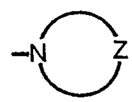

eine gesättigte 5-7gliedrige heterocyclische Gruppe ist, die zusätzliche N-, O- oder s-Atom(e) enthalten kann und einen bis drei Substituenten, ausgewählt aus niedrig-Alkyl-, niedrig-Alkylamino-, niedrig-Alkoxy-, Halogen-, Hydroxy-, Carboxy-, niedrig-Alkoxy-carbonyl- und Hydroxy- (niedrig)-alkyl-Gruppen haben kann.

3. Verbindung nach Anspruch 2, worin $R^1$ Alkoxy ist; $R^2$ niedrig-Alkoxy oder Ar-(niedrig)-Alkoxy ist; und A niedrig-Alkylen ist; und

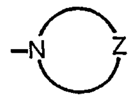

eine gesättigte 6- oder 7gliedrige heterocyclische Gruppe ist, die zusätzliche N- oder O-Atom(e) enthalten kann und 1 bis 3 Substituenten haben kann, ausgewählt aus niedrig-Alkyl, Hydroxy- und Hydroxy-(niedrig)-alkyl-Gruppen.

4. Verbindung nach Anspruch 3, worin $R^2$ niedrig-Alkoxy ist.

5. Verbindung nach Anspruch 4, worin

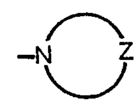

Morpholino, Piperazinyl, Piperidino oder Homopiperazinyl ist, das 1 bis 2 Substituenten haben kann, ausgewählt aus niedrig-Alkyl-, Hydroxy- oder Hydroxy-(niedrig)-alkylgruppen.

6. (rac)-1-O-Octadecyl-2-O-methyl-glycerin-3-[2-methyl-morpholinio)-ethyl]-phosphat.

7. (rac)-1-O-Hexadecyl-2-O-methyl-glycerin-3-[2-(4-methyl-1-piperazinyl)-ethyl]-phosphat.

8. (rac)-1-O-Octadecyl-2-O-methyl-glycerin-3-[2-(4-methyl-1-piperazinyl)-ethyl]-phosphat.

9. Verbindung nach Anspruch 3, worin $R^2$ Ar-(niedrig)-alkoxy ist.

10. (rac)-1-O-Hexadecyl-2-O-benzyl-glycerin-3-[2-(4-methyl-1-piperazinyl)-ethyl]-phosphat.

11. Verbindung nach Anspruch 1, worin

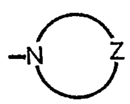

eine ungesättigte 5-7gliedrige heterocyclische Gruppe ist, die zusätzliche N-, O- oder S-Atom(e) enthalten kann und 1 bis 3 Substituenten haben kann, ausgewählt aus niedrig-Alkyl-, niedrig-Alkylamino-, niedrig-Alkoxy-, Halogen-, Hydroxy-, Carboxy-, niedrig-Alkoxycarbonyl- und Hydroxy-(niedrig)-alkylgruppen.

12. Verbindung nach Anspruch 11, worin $R^1$ Alkoxy mit 15 oder mehr Kohlenstoffatomen oder Alkenyloxy ist; $R^2$ Wasserstoff, Halogen, Hydroxy, niedrig-Alkoxy, Ar(niedrig)-alkoxy, Aryloxy, niedrig-Alkylthio, Arylthio, Thiadiazolylthio, niedrig-Alkylsulfonyl, niedrig-Alkoxycarbonylamino oder niedrig-Alkylureido ist; A niedrig-Alkylen, gegebenenfalls unterbrochen durch eine —NHCO—Gruppe ist; und

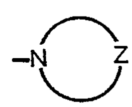

eine ungesättigte 5- oder 6gliedrige heterocyclische Gruppe ist, die zusätzliche N- oder S-Atom(e) enthalten kann und 1 oder 2 Substituenten haben kann, ausgewählt aus niedrig-Alkyl-, niedrig-Alkylamino-, Halogen-, niedrig-Alkoxycarbonyl- und Hydroxy-(niedrig)-alkyl-Gruppen.

13. Verbindung nach Anspruch 12, worin $R^1$ Alkoxy mit 15 oder mehr Kohlenstoffatomen ist.

14. Verbindung nach Anspruch 13, worin $R^2$ niedrig-Alkoxy ist.

15. (rac)-1-O-Octadecyl-2-O-methyl-glycerin-3-[2-(1-pyridinio)-ethyl]-phosphat.

16. (rac)-1-O-Octadecyl-2-O-methyl-glycerin-3-[2-(3-methyl-1-pyridinio)-ethyl]-phosphat.

17. (rac)-1-O-Octadecyl-2-O-methyl-glycerin-3-[2-(1-pyridinioacetylamino)-ethyl]-phosphat.

18. (rac)-1-O-Octadecyl-2-O-methyl-glycerin-3-[2-(4-methyl-1-pyridinio)-ethyl]-phosphat.

19. Verbindung nach Anspruch 13, worin $R^2$ Ar-(niedrig)-alkoxy ist.

20. (rac)-1-O-Hexadecyl-2-O-benzyl-glycerin-3-[2-(1-pyridinio)-ethyl]-phosphat.

21. Verbindung nach Anspruch 13, worin $R^2$ Hydroxy ist.

22. (rac)-1-O-Octadecyl-glycerin-3-[2-(1-pyridinio)-ethyl]-phosphat.

23. Verbindung nach Anspruch 13, worin $R^2$ Thiadiazolylthio ist.

24. (rac)-1-O-Octadecyl-2-(1,3,4-thiadiazol-2-ylthio)-1,3-propandiol-3-[2-(1-pyridinio)-ethyl]-phosphat.

25. Verbindung nach Anspruch 13, worin $R^2$ niedrig-Alkoxycarbonylamino ist.

26. (rac)-1-O-Octadecyl-2-ethoxycarbonylamino-1,3-propandiol-3-[2-(1-pyridinio)-ethyl]-phosphat.

27. Verfahren zur herstellung einer Verbindung der Formel (I), wie in Anspruch 1 beansprucht, und der pharmazeutische brauchbaren Salze davon durch

(1) Reaktion einer Verbindung der Formel

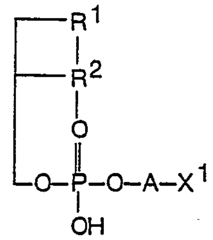

(II)

worin $R^1$, $R^2$ und A jeweils wie in Anspruch 1 definiert sind und $X^1$ ein Säurerest ist, oder ihres Salzes, mit einer Verbindung der Formel

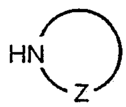

(III)

worin

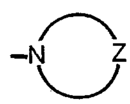

wie in Anspruch 1 definiert ist, oder ihres Salzes, zur Bildung einer Verbindung der Formel

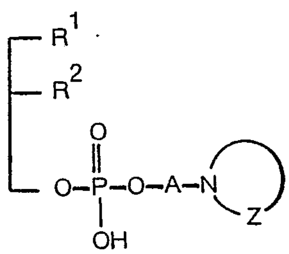

(I)

worin $R^1$, $R^2$, A und

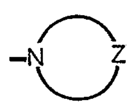

jeweils wie vorstehend definiert sind, oder ihres Salzes, oder

(2) Reaktion einer Verbindung der Formel

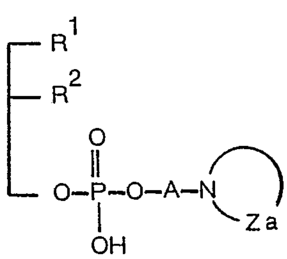

(Ib)

worin $R^1$, $R^2$ und A jeweils wie vorstehend definiert sind, und

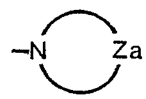

eine ungesättigte oder gesättigte 5-7-gliedrige heterocyclische Gruppe ist, die zusätzliche N-, O- oder S-Atom(e) enthalten kann, und 1 bis 2 Substituenten, ausgewählt aus niedrig-Alkyl-, niedrig-Alkylamino-, niedrig-Alkoxy-, Halogen-, Hydroxy-, Carboxy-, niedrig-Alkoxycarbonyl- und Hydroxy-(niedrig)-alkylgruppen, haben kann, oder ihres Salzes, mit einer Verbindung der Formel

$$X^2—R^3 \qquad (IV)$$

worin $R^3$ niedrig-Alkyl ist, und $X^2$ ein Säurerest ist, zur Bildung einer Verbindung der Formel

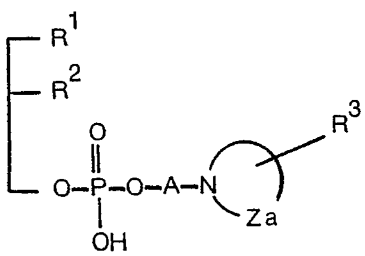

(Ia)

worin $R^1$, $R^2$, A, $R^3$ und

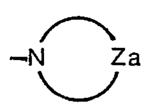

jeweils wie vorstehen definiert sind, oder ihres Salzes, oder

(3) Unterwerfen einer Verbindung der Formel

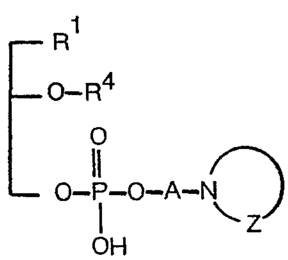

(V)

worin $R^1$ A und

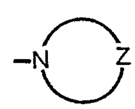

jeweils wie vorstehend definiert sind, und $R^4$ eine Schutzgruppe für Hydroxy ist, oder ihres Salzes, einer Eliminierungsreaktion für die Schutzgruppe, zur Bildung einer Verbindung der Formel

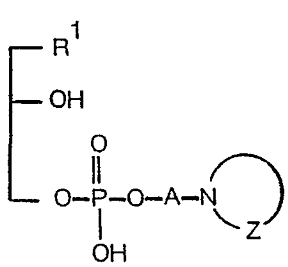

(Ic)

worin $R^1$, A und

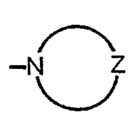

jeweils wie vorstehend definiert sind, oder ihres Salzes, oder

(4) Reaktion einer Verbinding der Formel

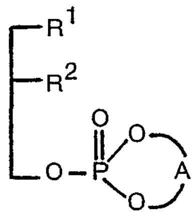

(VI)

worin $R^1$, $R^2$ und A jeweils wie vorstehend definiert sind, oder ihres Salzes mit einer Verbindung der Formel

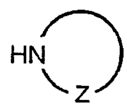

(III)

worin

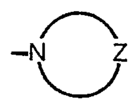

wie vorstehend definiert ist, oder ihrem Salz, zur Bildung einer Verbindung der Formel

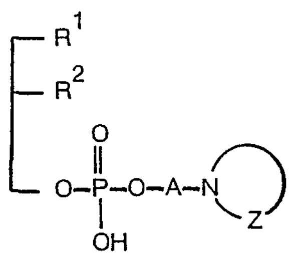

(I)

worin $R^1$, $R^2$, A und

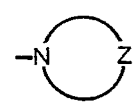

jeweils wie vorstehend sind, oder ihres Salzes.

28. Pharmazeutische Zusammensetzung, enthaltend eine wirksame menge einer Verbindung der Formel (I), wie in Anspruch 1 definiert, oder pharmazeutisch brauchbare Salze davon, mit einem pharmazeutish brauchbaren, im wesentlichen nicht-toxischen Träger oder Exzipienten.

29. Verbindung der Formel (I), wie in Anspruch 1 beansprucht, zur Verwendung als eine aktive therapeutische Substanz.

30. Verbindung der Formel (I), wie in Anspruch 1 beansprucht, zur Verwendung als eine aktive Substanz zur Bekämpfung von Krebs.

**Revendications**

1. Composé de formule:

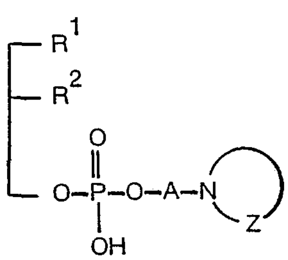

(I)

dans laquelle

$R^1$ est un groupe alkoxy ou alkénoxy en $C_1$ à $C_{25}$,

$R^2$ est l'hydrogène, un halogène, un groupe hydroxy, alkoxy inférieur, aryl-alkoxy inférieur, aryloxy, alkylthio inférieur, arylthio, thiadiazolylthio, alkylsulfonyl inférieur, alkoxy carbonyl amino inférieur ou alkyl uréido inférieur;

A est un groupe alkyène inférieur éventuellement interrompu par un groupe —NHCO—; et

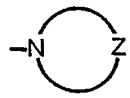

est un groupe hétérocyclique de 5 à 7 sommets, insaturé ou saturé, qui peut contenir en outre un (des) atome(s) de N, O ou S et peut comporter 1 à 3 substituants(s) choisi(s) parmi les groupes alkyle inférieur, alkylamino inférieur, alkoxy inférieur, halogène, hydroxy, carboxy, alkoxycarbonyle inférieur et hydroxyalkyle inférieur;

à condition que $R^1$ soit un groupe alkoxy ayant au moins 15 atomes de carbone lorsque

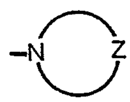

est un groupe pyridinio,
et leur sels pharmaceutiquement acceptables.

2. Composé selon la revendication 1, dans lequel

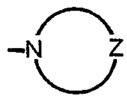

est un groupe hétérocyclique saturé de 5 à 7 sommets qui peut contenir en outre un (des) atome(s) de N, O ou S et peut comporter 1 à 3 substituant(s) choisi(s) parmi les groupes alkyle inférieur, alkylamino inférieur, alkoxy inférieur, halogène, hydroxy, carboxy, alkoxycarbonyle inférieur et hydroxyalkyle inférieur;

3. Composé selon la revendication 2, dans lequel $R^1$ est un groupe alkoxy;

$R^2$ est un groupe alkoxy inférieur ou aryl-alkoxy inférieur;

A est un groupe alkylène inférieur; et

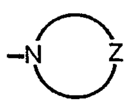

est un groupe hétérocyclique saturé de 6 ou 7 sommets qui peut contenir en outre un (des) atome(s) de N ou O et peut comporter 1 à 3 substituant(s) choisi(s) parmi les groupes alkyle inférieur, hydroxy et hydroxyalkyle inférieur.

4. Composé selon la revendication 3, dans lequel $R^2$ est un groupe alkoxy inférieur.

5. Composé selon la revendication 4, dans lequel

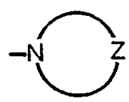

est un groupe morpholino, pipérazinyle pipéridino ou homopipérazinyle qui peut comporter 1 ou 2 substituant(s) choisi(s) parmi les groupes alkyle inférieur, hydroxy et hydroxyalkyle inférieur.

6. (rac)-1-O-Octadécyl-2-O-méthyl-glycérol-3-[2-(N-méthylmorpholinio)éthyl]phosphate.

7. (rac)-1-O-Hexadécyl-2-O-méthyl-glycérol-3-[2-(4-méthyl-1-pipérazinyl)éthyl]phosphate.

8. (rac)-1-O-Octadécyl-2-O-méthyl-glycérol-3-[2-(4-méthyl-1-pipérazinyl)éthyl]phosphate.

9. Composé selon la revendication 3, dans lequel $R^2$ est un groupe aryl-alkoxy inférieur.

10. (rac)-1-O-Hexadécyl-2-O-benzyl-glycérol-3-[2-(4-méthyl-1-pipérazinyl)éthyl]phosphate.

11. Composé selon la revendication 1, dans lequel

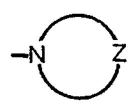

est un groupe hétérocyclique insaturé de 5 à 7 sommets qui peut contenir en outre un (des) atome(s) de N, O ou S et peut comporter 1 à 3 substituant(s) choisi(s) parmi les groupes alkyle inférieur, alkylamino inférieur, alkoxy inférieur, halogène, hydroxy, carboxy, alkoxycarbonyle inférieur et hydroxyalkyle inférieur.

12. Composé selon la revendication 11, dans lequel $R^1$ est un groupe alkoxý ayant au moins 15 atomes de carbone ou alkényloxy; $R^2$ est l'hydrogène, un halogène, un groupe hydroxy, alkoxy inférieur, aryl-alkoxy inférieur, aryloxy, alkylthio inférieur, arylthio, thiadiazolylthio, alkylsulfonyl inférieur, alkoxy carbonylamino inférieur ou alkyl uréido inférieur: A est un groupe alkylène inférieur éventuellement interrompu par un groupe —NHCO—; et

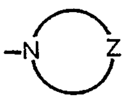

est un groupe hétérocyclique insaturé de 5 à 6 sommets qui peut contenir en outre un (des) atome(s) de N ou S et peut comporter 1 ou 2 substituant(s) choisi(s) parmi les groupes alkyle inférieur, alkylamino inférieur, halogène, alkoxycarbonyle inférieur et hydroxyalkyle inférieur.

13. Composé selon la revendication 12, dans lequel $R^1$ est un groupe alkoxy ayant ou moins 15 atomes de carbone.

14. Composé selon la revendication 13, dans lequel $R^2$ est un groupe alkoxy inférieur.

15. (rac)-1-O-Octadécyl-2-O-méthyl-glycérol-3-[2-(1-pyridinio)éthyl]phosphate.

16. (rac)-1-O-Octadécyl-2-O-méthyl-glycérol-3-[2-(3-méthyl-1-pyridinio)éthyl]phosphate.

17. (rac)-1-O-Octadécyl-2-O-méthyl-glycérol-3-[2-(1-pyridinioacétylamino)éthyl]phosphate.

18. (rac)-1-O-Octadécyl-2-O-méthyl-glycérol-3-[2-(4-méthyl-1-pyridinio)éthyl]phosphate.

19. Composé selon la revendication 13, dans lequel $R^2$ est un groupe aryl-alkoxy inférieur.

20. (rac)-1-O-Hexadécyl-2-O-benzyl-glycérol-3-[2-(1-pyridinio)éthyl]phosphate.

21. Composé selon la revendication 13, dans lequel $R^2$ est le groupe hydroxy.

22. (rac)-1-O-Octadécyl-glycérol-3-[2-(pyridinio)éthyl]phosphate.

23. Composé selon la revendication 13, dans lequel $R^2$ est le groupe thiadiazolylthio.

24. (rac)-1-O-Octadécyl-2-(1,3,4-thiadiazol-2-ylthio)-1,3-propane-diol-3-[2-(1-pyridinio)éthyl]phosphate.

25. Composé selon la revendication 13, dans lequel $R^2$ est un groupe alkoxy carbonylamino inférieur.

26. (rac)-1-O-Octadécyl-2-éthoxycarbonylamino-1,3-propane-diol-3-[2-(1-pyridinio)éthyl]phosphate.

27. Procédé de préparation d'un composé de formule (I) selon la revendication 1 et de ses sels pharmaceutiquement acceptables, qui comprend

(1) faire réagir un composé de formule:

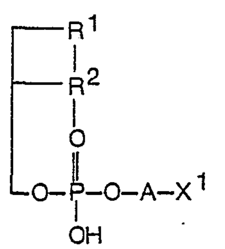

(II)

dans laquelle $R^1$, $R^2$ et A son chacun tels que définis dans la revendication 1 et $X^1$ est un radical acide, ou l'un de ses sels, avec un composé de formule

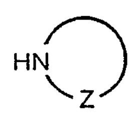

(III)

dans laquelle

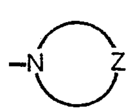

est tel que défini dans la revendication 1 ou l'un de ses sels pour donner un composé de formule:

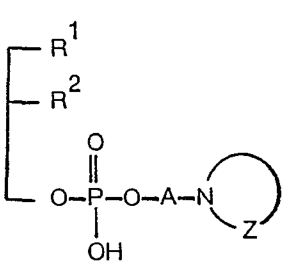

(I)

dans laquelle $R^1$, $R^2$, A et

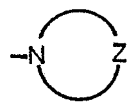

sont chacun tels que définis ci-dessus, ou l'un de ses sels, ou

(2) faire réagir un composé de formule:

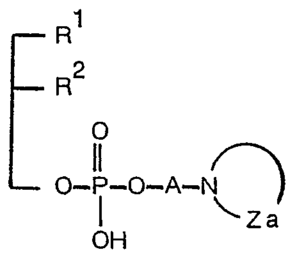

(Ib)

dans laquelle $R^1$, $R^2$ et A sont chacun tels que définis ci-dessus et

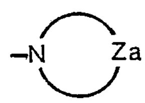

est un groupe hétérocyclique de 5 à 7 sommets, insaturé ou saturé, qui peut contenir en outre un (des) atome(s) de N, O ou S et peut comporter 1 à 2 substituant(s) choisi(s) parmi les groupes alkyle inférieur, alkylamino inférieur, alkoxy inférieur, halogène, hydroxy, carboxy, alkoxycarbonyle inférieur et hydroxyalkyle inférieur, ou un de ses sels, avec un composé de formule:

$$X^2—R^3 \qquad (IV)$$

dans laquelle $R^3$ est un groupe alkyle inférieur et $X^2$ est un radical acide, pour donner un composé de formule:

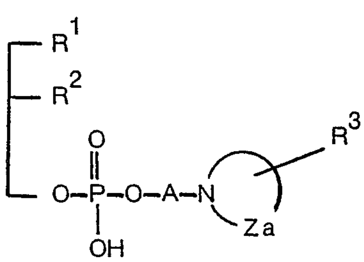

(Ia)

dans laquelle $R^1$, $R^2$, A, $R^3$ et

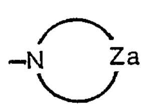

sont chacun tels que définis ci-dessus, ou un de ses sels, ou

(3) soumettre un composé de formule:

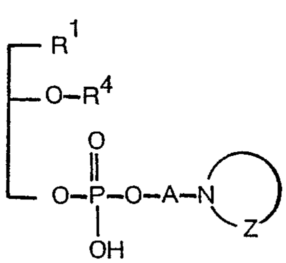

(V)

dans laquelle $R^1$, A et

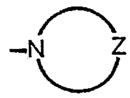

sont chacun tels que définis ci-dessus et $R^4$ est un groupe protecteur pour le groupe hydroxy, ou un de ses sels, à une réaction d'élimination du groupe protecteur pour donner un composé de formule:

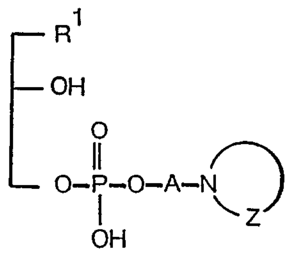

(Ic)

dans laquelle $R^1$, A et

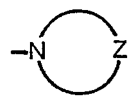

sont chacun tels que définis ci-dessus, ou un de ses sels, ou

(4) faire réagir un composé de formule:

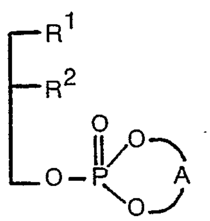

(VI)

dans laquelle $R^1$, $R^2$ et A son chacun tels que définis ci-dessus, ou un de ses sels, avec un composé de formule:

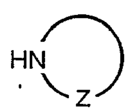

(III).

dans laquelle

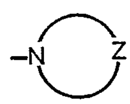

est tel que défini ci-dessus, ou un de sels pour donner un composé de formule:

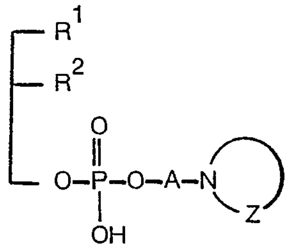

(I)

dans laquelle $R^1$, $R^2$, A et

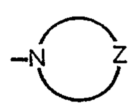

sont chacun tels que définis ci-dessus, ou un des ses sels.

28. Composition pharmaceutique comprenant une quantité efficace d'un composé de formule (I) selon la revendication 1 ou de ses sels pharmaceutiquement acceptables, avec un véhicule ou excipient essentiellement non toxique et pharmaceutiquement acceptable.

29. Composé de formule (I) selon la revendication 1, pour usage en tant que substance à activité thérapeutique.

30. Composé de formule (I) selon la revendication 1, pour usage en tant que substance active pour combattre le cancer.